# EUROPEAN PATENT SPECIFICATION

(11) **EP 3 308 699 B1**
(45) Date of publication and mention of the grant of the patent: **16.10.2019**
(21) Application number: 16807209.8
(22) Date of filing: 20.04.2016
(51) Int. Cl.: A61B 5/00, G08B 21/04, G08B 25/08, G06Q 10/06, H04L 29/08

(54) **CENTRAL PROCESSING DEVICE AND CENTRAL PROCESSING METHOD FOR SYSTEM FOR MONITORING PERSONS TO BE MONITORED, AND SYSTEM FOR MONITORING PERSONS TO BE MONITORED**
ZENTRALER PROZESSOR UND ZENTRALES VERARBEITUNGSVERFAHREN FÜR EIN SYSTEM ZUR ÜBERWACHUNG VON ZU ÜBERWACHENDEN PERSONEN UND SYSTEM ZUR ÜBERWACHUNG VON ZU ÜBERWACHENDEN PERSONEN
DISPOSITIF ET PROCÉDÉ DE TRAITEMENT CENTRAL POUR SYSTÈME DE SURVEILLANCE DE PERSONNES À SURVEILLER, ET SYSTÈME POUR SURVEILLER DES PERSONNES À SURVEILLER

(30) Priority: 09.06.2015 JP 2015116636
(43) Date of publication of application: 18.04.2018
(73) Proprietor: Konica Minolta, Inc., Tokyo 100-7015 (JP)
(72) Inventor: KAWAZU, Keiichi, Tokyo 100-7015 (JP)
(74) Representative: Gille Hrabal
(86) International application number: PCT/JP2016/062483
(87) International publication number: WO 2016/199502

(56) References cited:
- WO-A2-2007/081629
- JP-A- 2003 250 768
- JP-A- 2011 172 674
- JP-A- 2012 061 230
- US-A1- 2005 219 044
- US-A1- 2011 225 238

## Description

### Technical Field

The present invention relates to a central processing apparatus and a central processing method for use in a subject observation system for observing a subject as a watching target in cooperation with a plurality of devices, and a subject observation system.

### Background Art

Our country, Japan, has been experiencing an aging society, particularly, a super aging society where an aging rate of the population aged 65 and older to the total population exceeds 21 % because of improvements in living standards, sanitary conditions and medical levels accompanying with the rapid economic growth after the war. Also, the population aged 65 and older is expected to reach approximately 34,560,000 to the total population of 124,110,000 in 2020, while the population aged 65 and older was approximately 25,560,000 to the total population of approximately 127,650,000 in 2005. This aging society is expected to have a greater number of nursing or care needers (nursing needers or the like) due to illnesses, injuries or aging than a non-aging society. Moreover, our country also has been experiencing a declining-birth rate society, for example, the total fertility rate was 1.43 in 2013. This circumstance has also caused a problem of "care of an elderly person by another elderly person", which means that an elderly person who requires nursing or care has to be taken care of by an elderly family member such as a spouse, child, or sibling.

The nursing or care needers enter hospitals or facilities like welfare facilities (including short-stay facilities, care homes, intensive care homes, and the like referred by Japanese statutory laws) for the elderly, and receive nursing or care. These facilities face risks that nursing needers or the like get injuries by falling down from beds or falling over during walking, or loiter after sneaking out of the beds. In consideration that it is necessary to eliminate the risks as soon as possible and that the risks may lead to more serious problems if being left without any countermeasures, nurses and caregivers or the like confirm the safety or check the state of each of the nursing needers or the like through regular patrols.

However, the nursing and care industries encounter a problem of chronic labor shortage due to a slower increase in the number of nurses or the like than in the number of nursing needers or the like. Furthermore, compared with the day time, a workload per nurse or caregiver is much heavier during the semi-night time and the night time because of a decrease in the number of nurses or caregivers during those times. Hence, there has been a demand of reduction in the workload. Moreover, the aforementioned problem of "care of an elderly person by another elderly person" is seen in the facilities as well without exception, i.e., it is often recognized that an elderly nurse or the like has to take care of an elderly care needer or the like. Generally, as one gets older, his or her physical strength declines. This means that the nursing workload is harder for an older nurse than for a younger nurse regardless of his or her good health, and the older nurse is considered to delay in movement or judgment.

In order to alleviate the labor shortage and the workload of the nurses or the like, technologies of supporting the nursing and caring workloads have been demanded. In response to this demand, subject observation apparatuses have been recently researched and developed to observe (monitor) a subject as a watching target, such as a nursing needer or the like, to be observed.

The technologies involve an exemplary call system disclosed in Patent Literature 1. The call system disclosed in Patent Literature 1 is installed in a facility having a plurality of resident rooms, and includes a plurality of call terminals respectively allotted to residents living in the resident rooms, mobile terminals carried by facility staffs who perform nursing or care of the residents, and an administration server for controlling and administrating information about a facility staff call sent and received between the call terminals and the mobile terminals, the call terminals, the mobile terminals and the administration server being connected to a facility intra-network to thereby establish the call system. Each of the call terminals includes a call terminal display having a touch panel function, button display control means for displaying on the call terminal display a plurality of request buttons representing requests to call facility staffs, and call information sending means for sending to the administration server via the facility intra-network call information corresponding to one of the request buttons together with terminal identification information peculiar to the call terminal upon inspection of a call operation that the one button is touched by means of the touch panel function. The administration server includes: resident registration means for storing and registering resident information containing names of the residents and room numbers of the resident rooms in association with terminal identification information peculiar to the call terminal allotted to each of the residents; staff registration means for storing and registering staff information containing information about names of the facility staffs and the residents or the resident rooms for which the facility staffs are responsible in association with mobile identification information peculiar to the mobile terminals carried by the facility staffs; call information receiving means for receiving the call information sent from the call terminal; mobile terminal extracting means for specifying the call terminal having sent the received call information in connection with the terminal identification information, and extracting one of the mobile terminals carried by corresponding one of the facility staffs who is responsible for the resident allotted with the call terminal or the corresponding resident room in connection with the staff information; and call information transfer means for transferring to the extracted mobile terminal via the facility intra-network the call information accompanied by the resident information of the resident allotted with the call terminal having sent the call information. Each of the mobile terminals includes a mobile terminal display, call information transfer receiving means for receiving the transferred call information and the resident information through a wireless communication line of the facility intra-network, and mobile display control means for displaying the received call information and the resident information on the mobile terminal display. Moreover, in the call system, the call terminal further includes calling display control means for displaying on the call terminal display a state that one of the facility staffs is being called after sending the call information, and call accepting display control means for displaying on the call terminal display a state that the call is being accepted when the administration server receives confirmation information indicating that the facility staff goes to the resident room of the resident from the mobile terminal to which the call information has been transferred from the administration server. The administration server further includes confirmation information receiving means for receiving the confirmation information from the mobile terminal. The mobile terminal further includes confirmation input and sending means for accepting an input of confirmation information indicating that the facility staff having confirmed contents of the received call information goes to the resident room, and sending the input confirmation information to the administration server.

Meanwhile, a person living alone is also a subject to be observed as well as the nursing needer or the like in terms of safety confirmation.

In the call system disclosed in Patent Literature 1, when the call terminal accepts the call operation, the administration server sends the call information to the mobile terminal of the facility staff who is responsible for the resident room corresponding to the call terminal, the mobile terminal displays the call information. The mobile terminal notifies the administration server that the facility staff goes to the resident room upon receipt of the information about the going of the facility staff.

By the way, a plurality of nurses or the like normally work in facilities such as hospitals and welfare facilities for the elderly, and they respectively carry mobile terminals. In the call system disclosed in Patent Literature 1, the call terminals, the administration server and the mobile terminals send and receive information therebetween, and are coordinated with one another. However, the plurality of mobile terminals are not coordinated with each other. Hence, if the call system disclosed in Patent Literature 1 is applied to a system for sending to the plurality of mobile terminals a notification about a nursing needer or the like, one nurse or the like carrying a corresponding one of the mobile terminals can respond to the nursing needer or the like, but another nurse carrying another mobile terminal cannot recognize the responding state of the one nurse. Thus, this may cause a situation that two or more nurses or the like rush to one nursing needer to respond thereto, or another situation that none of the nurses responds to the nursing needer due to the lack of recognition about the states of other nurses.

### Citation List

### Patent Literature

Patent Literature 1: Japanese Unexamined Patent Publication JP2014090913.

Document WO2007/081629 A2 discloses a method and system for monitoring a person requiring care by designated caregivers, for instance in medical structures. In case that assistance is needed, one caregiver at a time is alerted and, in case of no response within a time limit, a different caregiver is notified.

### Summary of Invention

The present invention has been accomplished in view of the above-described situations. An object of the present invention is to provide a central processing apparatus and a central processing method for use in a subject observation system which render terminal devices to coordinate with one another, and a subject observation system.

A central processing apparatus and a central processing method for use in a subject observation system, and a subject observation system according to the present invention include: storing an associative relationship between a sensor unit for inspecting observational information about a subject to be observed and a group composed of a specified number of terminal devices for receiving and displaying the observational information via the central processing apparatus based on the inspection result; and, when receiving from a first terminal device a first acceptance notification signal having received to notify the central processing apparatus that a predetermined operation to the sensor unit is accepted by the first terminal device, sending to a second terminal device a second acceptance notification signal indicating that the predetermined operation is accepted by the first terminal device, the second terminal device belonging to the first group to which the first terminal device belongs and other than the first terminal device. In this manner, the central processing apparatus and the central processing method for use in the subject observation system, and the subject observation system can render the terminal devices coordinate with each other.

These and other subjects, features and advantages of the present invention will become more apparent upon reading the following detailed description along with the accompanying drawings.

### Brief Description of Drawings

FIG. 1 shows a configuration of a subject observation system according to an embodiment.
FIG. 2 shows a configuration of an administration server for use in the subject observation system.
FIG. 3 shows a configuration of an observational information table stored in the administration server.
FIG. 4 shows a configuration of a group information table stored in the administration server.
FIG. 5 shows a configuration of a terminal information table stored in the administration server.
FIG. 6 shows a configuration of a sensor information table stored in the administration server.
FIG. 7 shows a configuration of a subject information table stored in the administration server.
FIG. 8 shows a configuration of a mobile terminal device in the subject observation system.
FIG. 9 is a flowchart showing an operation of the mobile terminal device for receiving notification of observational information.
FIG. 10 shows an exemplary standby screen image displayed on the mobile terminal device.
FIG. 11 shows an exemplary observational information screen image displayed on the mobile terminal device having received a notification of waking-up movement of a subject.
FIG. 12 is a flowchart showing an operation of the administration server for coordinating mobile terminal devices.
FIG. 13 is a flowchart showing an operation of the mobile terminal device for following the coordination with other mobile terminal devices.
FIG. 14 shows a sequence diagram illustrating an exemplary coordination between two mobile terminal devices.
FIG. 15 shows a diagram illustrating screen image shifts in connection with an exemplary coordination between two mobile terminal devices.
FIG. 16 is a flowchart showing an operation of the administration server for sending observational information to a second group.

### Description of Embodiments

Hereinafter, an embodiment of the present invention will be described with reference to the accompanying drawings. Elements denoted by the same reference numerals in the drawings have the same configuration and, therefore, repeated descriptions will be appropriately omitted. In the present specification, elements are denoted by a same reference numeral when being referred to collectively, and are denoted by a same reference numeral accompanied by a different respective reference character when being referred to individually.

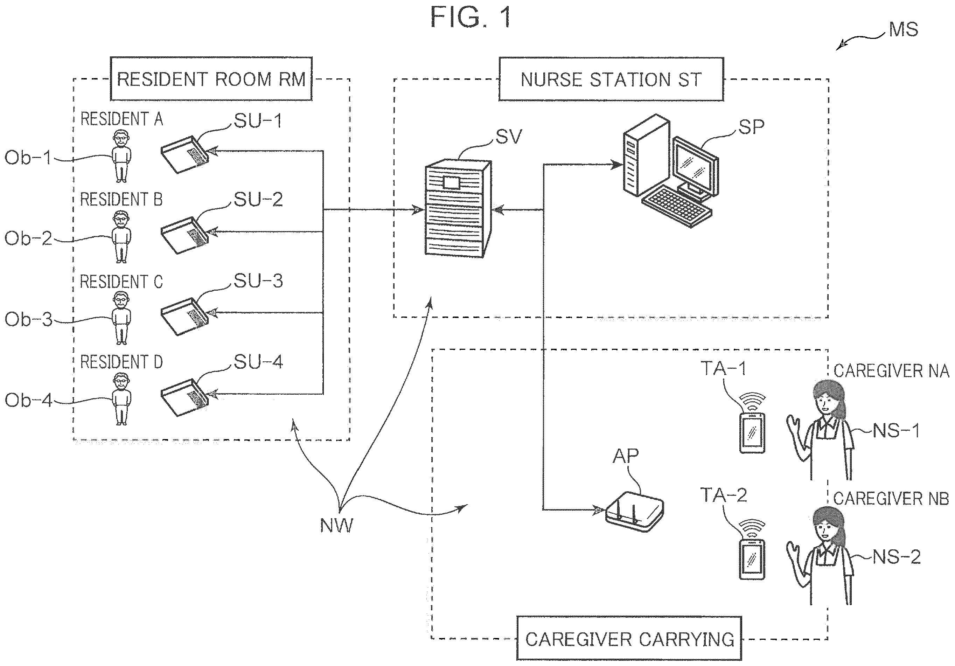

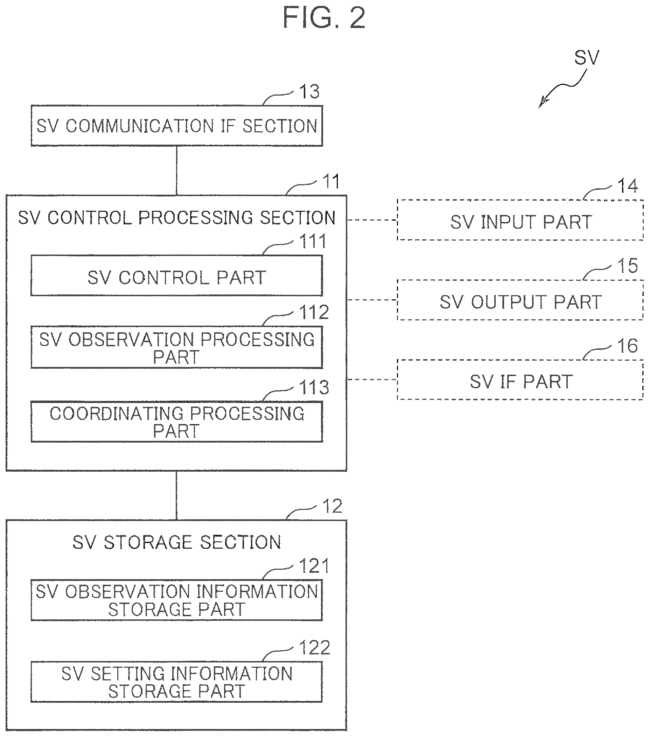

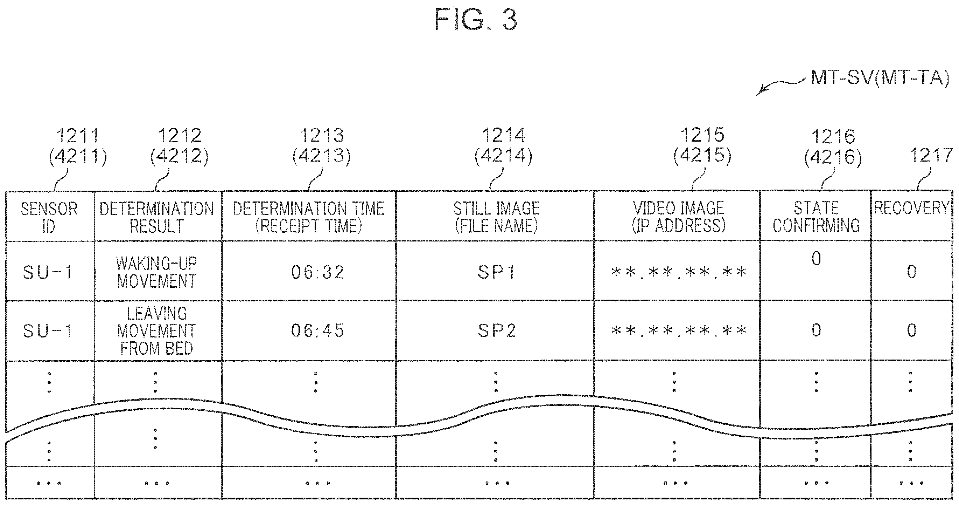

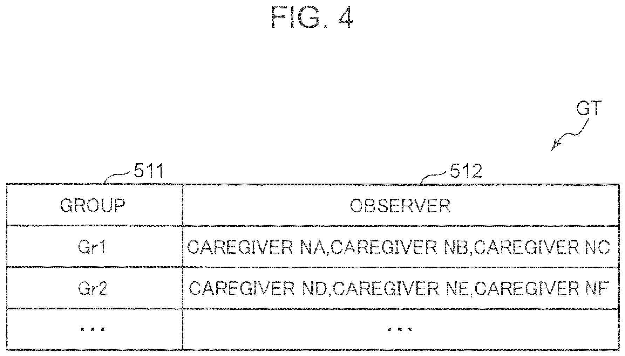

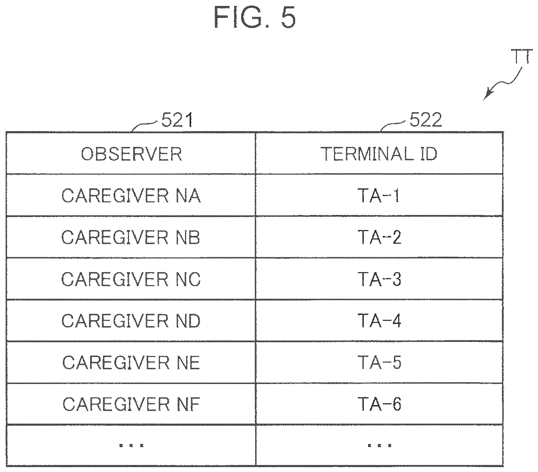

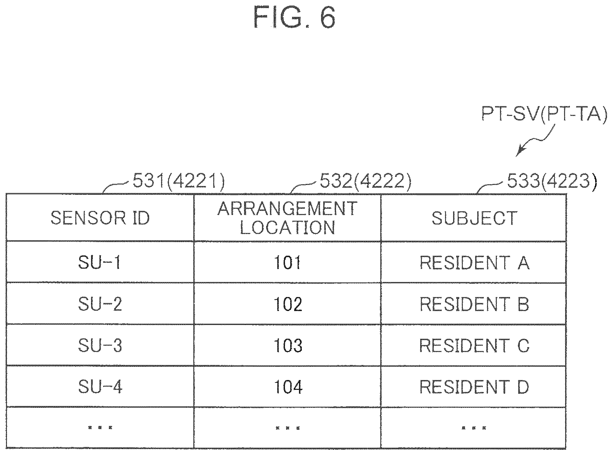

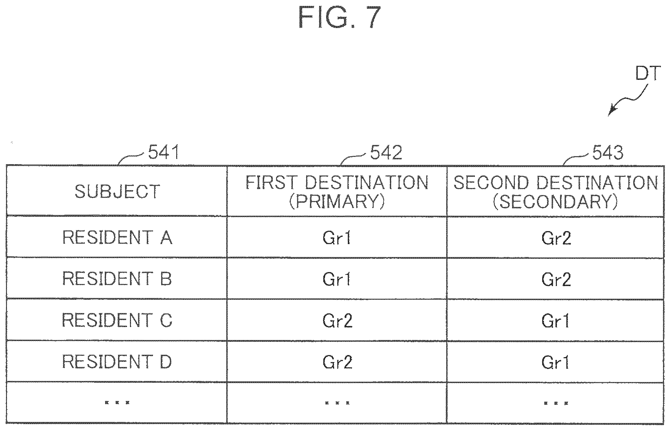

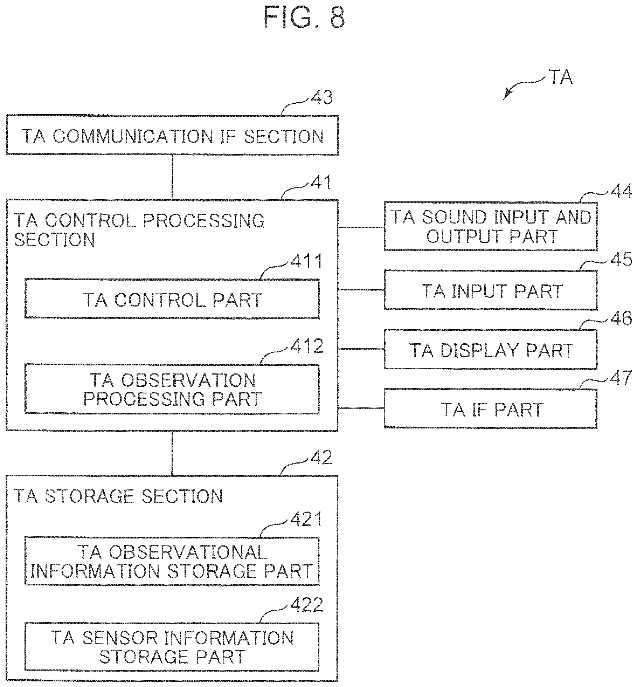

FIG. 1 shows a configuration of a subject observation system according to an embodiment. FIG. 2 shows a configuration of an administration server for use in the subject observation system. FIG. 3 shows a configuration of an observational information table stored in the administration server. FIG. 4 shows a configuration of a group information table stored in the administration server. FIG. 5 shows a configuration of a terminal information table stored in the administration server. FIG. 6 shows a configuration of a sensor information table stored in the administration server. FIG. 7 shows a configuration of a subject information table stored in the administration server. FIG. 8 shows a configuration of a mobile terminal device in the subject observation system.

A subject observation system watches a subject Ob as a watched target (supervised target) to be observed (supervised) by a plurality of devices to thereby accomplish observation of the subject Ob. For example, as shown in FIG 1, a subject observation system MS includes one or more sensor units (inspector units) SU (SU-1 to SU-4), an administration server SV, a stationary terminal device SP and one or more mobile terminal devices TA (TA-1, TA-2), which are communicatively connected with one another via a wired or wireless network NW or communication line, such as a LAN (Local Area Network), a telephone network and a data communication network. The network NW may include a relay device, such as a repeater, bridge, rooter and cross connect, to relay a communication signal. In the embodiment shown in FIG 1, the sensor units SU-1 to SU-4, the administration server SV, the stationary terminal device SP and the mobile terminal devices TA-1, T1-2 are communicatively connected with one another via a wireless LAN (such as a LAN satisfying IEEE802.11 standard) NW including an access point AP.

The subject observation system MS is disposed at a location suitable for the subject Ob. The subject (supervising target) Ob may involve, for example, a person who requires nursing due to an illness or injury, a person who needs care due to reduction in the physical ability, and a person living alone. Particularly, the subject Ob is appreciated to require finding of a predetermined inconvenient incident, for example, an abnormality condition, happened to the subject in terms of achievement in early finding and quick response. For this reason, the subject observation system MS is preferably disposed in a building, such as a hospital, a welfare facility for the elderly and a house, depending on a type of the subject Ob. In the embodiment shown in FIG. 1, the subject observation system MS is disposed in a care facility building provided with a plurality of chambers including resident rooms RM respectively for a plurality of subjects Ob to live therein, a nurse station ST, and other rooms.

Each of the sensor units SU has a communication function to communicate with the other devises SV, SP, TA via the network NW, and serves as a device for generating an image by photographing the subject Ob and inspecting the subject Ob on the basis of the generated image. Specifically, the sensor unit SU is, for example, composed of: a communication interface circuit, such as an LAN card or the like, to communicate with the devices SV, SP, TA via the network NW; a body motion sensor of a doppler shift type for inspecting the subject Ob by detecting a doppler shift of a microwave generated due to a body motion like breathing by the subject Ob after sending or receipt of a microwave; an image sensor for photographing the subject Ob and generating an image; a data processing circuit for determining a state (condition) of the subject Ob as an inspection result of the subject Ob in connection with an output from the body motion sensor (body motion sensor output) and an output (image) from the image sensor; a control circuit for controlling all the aforementioned elements; and peripheral circuits therearound. The sensor unit SU sends the inspection result to the administration server SV. The sensor unit SU sends the generated image (including a still image and a video image) to the predetermined devices SV, SP, TA. The embodiment shown in FIG. 1 includes first through fourth sensor units SU-1 through SU-4. The first sensor unit SU-1 is arranged in an unillustrated resident room RM-1 of a resident A, i.e., subject Ob-1, who is one of the subjects Ob, the second sensor unit SU-2 is arranged in an unillustrated resident room RM-2 of another resident B, i.e., subject Ob-2, who is another one of the subjects Ob, the third sensor unit SU-3 is arranged in an unillustrated resident room RM-3 of further another resident C, i.e., subject Ob-3, who is further another one of the subjects Ob, and the fourth sensor unit SU-4 is arranged in an illustrated resident room RM-4 of sill further another resident D, i.e., subject Ob-4, who is one of the subjects Ob.

The stationary terminal device SP has a communication function to communicate with the other devices SU, SV, TA via the network NW, a display function to display predetermined information, and an input function to input a predetermined instruction or predetermined data, and further serves as a user interface (UI) of the subject observation system MS by inputting the predetermined instruction or data to be given to the administration server SV or the mobile terminal device TA, displaying the inspection result or image obtained at the sensor unit SU and the like. For example, the stationary terminal device SP may be made up by a computer having a communication function.

The administration server SV has a communication function to communicate with the other devices SU, SP, TA via the network NW, and serves as a device for administrating observational information about observation of the subject Ob by receiving from the sensor unit SU the inspection result about the subject. As shown in FIG. 2, the administration server SV includes, for example, a server control processing section (SV control processing section) 11, a server storage section (SV storage section 12), and a server communication interface section (SV communication IF section) 13. The administration server SV is an exemplary central processing apparatus.

The SV communication IF section 13 is connected to the SV control processing section 11, and serves as a circuit for performing a communication in accordance with a control of the SV control processing section 11. The SV communication IF section 13 generates a communication signal containing data input from the SV control processing section 11 for transfer in accordance with a communication protocol used in the network NW of the subject observation system MS, and sends the generated communication signal to the other devices SU, SP, TA via the network NW. The SV communication IF section 13 receives a communication signal from the devices SU, SP, TA via the network NW, takes data from the received signal, converts the taken data to another one having a different format that can be processed by the SV control processing section 11, and outputs the converted data to the SV control processing section 11.

The SV storage section 12 is connected to the SV control processing section 11, and serves as a circuit for storing various predetermined programs and data. For example, the various predetermined programs include control processing programs such as a server program for providing a client (the stationary terminal device SP and the mobile terminal device TA in the embodiment) with data in response to a request of the client, and an observational processing program for processing the observational information about the observation of the subject Ob. The observational processing program further involves a coordinative processing program for coordinating the plurality of terminal devices (the stationary terminal device SP and the mobile terminal device TA in the embodiment shown in FIG. 1, here, it should be noted that the term "terminal devices SP, TA" correspond to the stationary terminal device SP and the mobile terminal device TA). The various predetermined data includes: a communication address (such as an IP address) of each of the terminal devices SP, TA; the observational information about the observation of the subject Ob; group information about a group of the mobile terminal devices TA to which the observational information from the sensor unit SU should be sent; terminal information about the mobile terminal device TA; sensor information about the sensor unit SU; and subject information about the subject Ob. The group information may include information of the stationary terminal device SP in addition to that of the mobile terminal device TA. The SV storage section 12 includes, for example, a ROM (Read Only Memory), an EEPROM (Electrically Erasable Programmable Read Only Memory) which is a rewritably nonvolatile storage element, and the like. The SV storage section 12 further includes a RAM (Random Access Memory) serving as a working memory of the SV control processing section 11 to store data generated during execution of the predetermined programs.

The SV storage section 12 is operably provided with a server observational information storage part (SV observational information storage part) 121 for storing the observational information, and a server setting information storage part (SV setting information storage part) 122 for storing the group information, the terminal information, the sensor information and the subject information as setting information.

The SV observational information storage part 121 stores the observational information about the observation of the subject Ob. The observational information is information about the subject Ob from the sensor unit SU and the terminal devices SP, TA, and includes: a sensor unit identifier (sensor ID) to specify and identify the sensor unit SU; information corresponding to the sensor ID and representing a determination result that a state of the subject Ob is determined by the sensor unit SU having the sensor ID (determination result information in the embodiment includes: waking-up movement; leaving movement from a bed; falling down or over; and abnormally irregular motion); information (determination time information) corresponding to the sensor ID and representing a time at which the state of the subject Ob is determined by the sensor unit SU having the sensor ID; a still image (or one of plural images used for the determination, for example, the last image) of the subject Ob corresponding to the sensor ID and used for the determination of the state of the subject Ob by the sensor unit US having the sensor ID; a communication address (such as an IP address) corresponding to the sensor ID and allotted to the sensor unit SU having the sensor ID; state confirming information corresponding to the sensor ID and representing whether or not the state of the subject Ob observed by the sensor unit SU having the sensor ID is being confirmed by the terminal devices SP, TA; and recovery information (response information) representing whether or not there is a fact or will of actual execution of a response, such as, lifesaving, nursing or care is inputted to the terminal devices SP, TA. In this case, a receipt time of the communication signal may be appreciated to be adopted in place of the determination time, the communication signal indicating that the determination result and image have been received.

In the embodiment, the observational information is stored in a table format in the SV observational information storage part 121. As shown in FIG. 3, a server observational information table (SV observational information table) MT-SV where the observational information is registered, for example, includes: a sensor ID field 1211 for registering the sensor ID; a determination result field 1212 for registering the determination result information about the sensor unit SU corresponding to the sensor ID registered in the sensor ID field 1211; a determination time field 1213 for registering the determination time information about the sensor unit SU corresponding to the sensor ID registered in the sensor ID field 1211; a still image field 1214 for registering the still image about the sensor unit SU corresponding to the sensor ID registered in the sensor ID field 1211; a video image field 1215 for registering a communication address (such as an IP address) of the sensor unit SU corresponding to the sensor ID registered in the sensor ID field 1211; a state confirming field 1216 for registering the state confirming information about the subject Ob observed by the sensor unit SU corresponding to the sensor ID registered in the sensor ID field 1211; and a recovery field 1217 for registering the recovery information about the subject Ob observed by the sensor unit SU corresponding to the sensor ID registered in the sensor ID field 1211. The SV observational information table MT-SV further has a record per observational information communication signal from the sensor unit SU. In the state confirming field 1216, registered as state confirming information representing whether or not the state is being confirmed is a terminal identifier (terminal ID) of each of the terminal devices SP, TA confirming the state of the subject Ob during confirmation of the state of the subject Ob, while a flag "0" representing non-confirming where the state of the subject Ob is left unconfirmed is registered during the non-confirming. The terminal ID is used to specify and identify the stationary terminal device SP and the mobile terminal device TA. In the recovery field 1217, registered is a flag representing the recovery information showing recovery or non-recovery. For example, in the recovery field 1217 of the embodiment, registered is a flag "1" representing an input of a fact or will of actual execution of a response (hereinafter, appropriately referred to as "a fact or the like of a response") to the terminal devices SP, TA, or a flag "0" representing no input of the fact or the like of the response to the terminal devices SP, TA. Also, as default, in the state confirming field 1216, registered is a flag "0" representing the non-confirming, and in the recovery field 1217, registered is a flag "0" representing no input of the fact or the like of the response. In the still image field 1214, it is appreciate to register, for example, image data of the still image, and further a file name of the image data of the still image. FIG 3 shows an example where a first record represents "SU-1", "waking-up movement", "06:32", "SP1", "**.**.**.** (here, it should be noted that denoted at the sign "**" is an integer value)", "0" and "0" registered respectively in the sensor ID field 1211, the determination result field 1212, the determination time field 1213, the still image field 1214, the video image field 1215, the state confirming field 1216, and the recovery field 1217.

Furthermore, in the example shown in FIG. 3, the server observational information table MT-SV includes the video image field 1215. However, it is appreciated to store another table in the SV observational information storage part 121 in advance in addition to the SV observational information table MT-SV, another table showing an associative relationship between the sensor ID and the communication address of the sensor unit SU as an acquisition destination of a live video image. In this case, the video image field 1215 may be excluded from the SV observational information table MT-SV shown in FIG. 3. The sensor ID may be in the form of a communication address such as, a MAC (Media Access Control) address, an IP (Internet Protocol) address or the like. In this case, it is appreciated to exclude the video field 1215 and the associative relationship. Similarly, the terminal ID also may be in the form of a communication address.

The SV setting information storage part 122 stores the group information, the terminal information, the sensor information and the subject information as the setting information. The group information represents, for example, an associative relationship between a group to which the observational information about the subject Ob in connection with the inspection result from the sensor unit SU is sent and an observer (like a caregiver in the embodiment) belonging to the group. The terminal information represents, for example, an associative relationship between the observer and the mobile terminal device TA carried by the observer. The sensor information represents, for example, an associative relationship among the sensor unit SU, an arrangement location of the sensor unit SU, and the subject Ob to be observed by means of the sensor unit SU. The subject information represents, for example, an associative relationship among the subject Ob, a first group as a first destination to which the observational information about the subject Ob in connection with the inspection result from the sensor unit SU inspecting the subject Ob is sent, and a second group as a second destination, the observational information being sent to the second group next to the first group. As an example in the embodiment, the associative relationship with the first group composed of a specified number of mobile terminal devices TA that are first destinations to which the observational information about the subject Ob in connection with the received inspection result from the sensor unit SU is sent, and the associative relationship with the second group composed of another specified number of mobile terminal devices TA that are the second destinations are established on the basis of the group information, the terminal information, the sensor information, and the subject information, the observational information being sent to the second group next to the first group.

In the embodiment, each of the group information, the terminal information, the sensor information, and the subject information is stored in a table format in the SV setting information storage part 122.

As shown in FIG. 4, a group information table GT where the group information is registered includes, for example, a group field 511 for registering a group ID as an identifier to specify and identify a group, and an observer field 512 for registering a name of an observer belonging to a group corresponding to the group ID registered in the group field 511, and further has a record per group ID. FIG. 4 shows an example where the first record represents "Gr1" and "caregiver NA, caregiver NB, and caregiver NC" respectively registered in the group ID field 511 and the observer field 512. The group having the group ID of "Gr 1" is composed of the caregivers NA, NB, NC belonging thereto.

As shown in FIG. 5, a terminal information table TT where the terminal information is registered includes, for example, an observer field 521 for registering a name of an observer, and a terminal ID field 522 for registering a terminal ID of the mobile terminal device TA used by the observer registered in the observer field 521, and further has a record per observer. FIG. 5 shows an example where a first record represents "caregiver NA" and "TA-1" respectively registered in the observer field 521 and the terminal ID field 522. The caregiver NA logs in the mobile terminal device TA-1 having the terminal ID of "TA-1" to thereby use it.

As shown in FIG 6, a server sensor information table (SV sensor information table) PT-SV where the sensor information is registered includes, for example, a sensor ID field 531 for registering the sensor ID, an arrangement location field 532 for registering an arrangement location of the sensor unit SU corresponding to the sensor ID registered in the sensor ID field 531, a subject field 533 for registering a name of the subject Ob to be observed by means of the sensor unit SU corresponding to the sensor ID registered in the sensor ID field 531, and further has a record per sensor ID. FIG 6 shows an example where a first record represents "SU-1", "101" and "resident A" respectively registered in the sensor ID field 531, the arrangement location field 532 and the subject field 533. The sensor unit SU-1 having the sensor ID of "SU-1" is arranged in resident room 101, and inspects the resident A.

As shown in FIG. 7, a subject table DT where the subject information is registered includes, for example, a subject field 541 for registering a name of the subject Ob, a first destination field (primary field) 542 for registering a group ID of the first group as the first destination to which the observational information about the subject Ob in connection with the inspection result from the sensor unit SU inspecting the subject Ob in the subject field 541 is sent, and a second destination field (secondary field) 543 for registering a group ID of the second group as the second destination to which the observational information about the subject Ob in connection with the inspection result from the sensor unit inspecting the subject Ob in the subject field 541 is sent, the observational information being sent to the second group next to the first group, and the subject table DT further has a record per the subject Ob. FIG. 7 shows an example where a first record represents "resident A", "Gr1" and "Gr2" respectively registered in the subject field 541, the first destination field 542 and the second destination field 543. The observational information from the sensor unit SU-1 inspecting the subject of "resident A" is first sent to the mobile terminal device TA used by an observer belonging to one group (corresponding to the first group) having the group ID of "Gr1", and is next sent to the mobile terminal device TA used by another observer belonging to another group (corresponding to the second group) having the group ID of "Gr2" when a predetermined condition is satisfied.

Here, each of the first and second groups may include the stationary terminal device SP in addition to the mobile terminal device TA. Particularly, the group 2 is appreciated to include the stationary terminal device SP in order to avoid delay or failure in response.

The SV control processing section 11 serves as a circuit for controlling respective parts of the administration server SV in accordance with their functions, and administrating the observational information of the subject Ob in connection with the inspection result about the subject Ob from the sensor unit SU. The SV control processing section 11 includes, for example, a CPU (Central Processing Unit) and peripheral circuits therearound. The SV control processing section 11 is operably provided with a server control part (SV control part) 111, a server observation processing part (SV observation processing part) 112 and a coordinating processing part 113 owing to execution of the control processing programs.

The SV control section part 111 controls the respective parts of the administration server SV in accordance with their functions to thereby control the entirety of the administration server SV.

The SV observation processing part 112 administrates the observational information. Specifically, in the embodiment, for example, when the SV communication IF section 13 receives from the sensor unit SU an observational information communication signal to be described later, the SV observation processing part 112 causes the SV observational information storage part 121 to store the information contained in the received observational information communication signal by registering the information in the SV observational information table MT-SV, and further renders the SV communication IF section 13 to send to the terminal devices SP, TA the observational information communication signal after a necessary modification is made as described later. If necessary, the SV observation processing part 112 updates the sate confirming field 1216 and the recovery field 1217 by rewriting them during the sending or receiving performance of the communication signal.

When the SV communication IF section 13 receives from one mobile terminal device TA a communication signal (i.e., first acceptance notification signal) having been sent to notify the administration server SV that a predetermined operation to the sensor unit SU is accepted by the one mobile terminal device TA, the coordinating processing part 113 renders the SV communication IF section 13 to send to a second mobile terminal device TA another communication signal (i.e., second acceptance notification signal) indicating that the predetermined operation is accepted by the one mobile terminal device TA, the second terminal device belonging to the first group to which the one mobile terminal device belongs and other than the one mobile terminal device TA. The coordinating processing part 113 renders the SV communication IF section 13 to further send to the second mobile terminal device TA a further another communication signal (i.e., acceptance prohibition signal) indicating that the second mobile terminal device TA is prohibited from accepting the predetermined operation. The coordinating processing part 113 renders the SV communication IF section 13 to further send to the second mobile terminal device TA a still further another communication signal (i.e., response display communication signal) of causing the second mobile terminal device TA to display a message stating that the one mobile terminal device TA is responding. In the embodiment, the acceptance prohibition signal is used as the second acceptance notification signal, and the response display communication signal is used as the acceptance prohibition signal as well. In other words, in the embodiment, the second acceptance notification signal functions as the acceptance prohibition signal and the response display communication signal. Moreover, when the SV communication IF section 13 receives no first acceptance notification signal until a predetermined time period (for example, five, ten or twenty minutes, or the like) lapses from a timing at which the SV communication IF section 13 sends to the mobile terminal devices TA in association with the sensor unit SU and belonging to the first group the observational information about the subject Ob in connection with the inspection result from the sensor unit SU, the coordinating processing part 113 renders the SV communication IF section 13 to send to the mobile terminal devices TA belonging to the second group the observational information about the subject Ob in connection with the received inspection result from the sensor unit SU.

As shown by the dashed line in FIG. 2, the administration server SV may be appreciated to include, for example, a server input part (SV input part) 14 for inputting various commands and data, a server output part (SV output part) 15 for outputting the various commands and data input by the SV input part 14 and information about the observation of the subject Ob and the like, and a server interface part (SVIF part) 16 for performing the input and output of the data in cooperation with the external devices, the parts 14, 15 and 16 being connected to the SV control processing section 11.

The administration server SV may be made up by, for example, a computer having a communication function.

Next, the mobile terminal device TA will be described. The mobile terminal device TA has a communication function to communicate with the other devices SV, SP, SU via the network NW, a display function to display predetermined information, an input function to input a predetermined instruction or predetermined data, and a talking function to perform a voice communication, and serves as a device for receiving and displaying the observational information about the observation of the subject Ob by inputting the predetermined instruction or data to be given to the administration server SV or the sensor unit SU, and displaying the inspection result or image obtained by the sensor unit SU by means of notification from the administration server SV.

As shown in FIG. 8, in the embodiment, the mobile terminal device TA includes, for example, a terminal control processing section (TA control processing section) 41, a terminal storage section (TA storage section) 42, a terminal communication interface section (TA communication IF section) 43, a terminal sound input and output part (TA sound input and output part) 44, a terminal input part (TA input part) 45, and a terminal display part (TA display part) 46, and a terminal interface part (TAIF part) 47.

The TA sound input and output part 44 is connected to the TA control processing section 41, and serves as a circuit for acquiring an external sound and inputting the acquired sound into the mobile terminal device TA, and further generating and outputting a sound corresponding to a sound representative electric signal in accordance with control by the TA control processing section 41. The TA sound input and output part 44 includes, for example, a microphone to convert a sound acoustic vibration to the electric signal, and a speaker to convert a sound electric signal to a sound acoustic vibration, and other elements. The TA sound input and output part 44 outputs an external sound representative electric signal to the TA control processing section 41, and converts the electric signal input from the TA control processing section 41 to the sound acoustic vibration to thereby output the converted sound acoustic vibration.

The TA input part 45 is connected to the TA control processing section 41, and serves as a device, such as a plurality of switches allotted with predetermined functions, for accepting a predetermined operation and inputting it into the mobile terminal device TA. The predetermined operation involves various operations necessary for the observation, for example, an operation of inputting an ID for logging in, another operation of inputting an instruction for displaying an image photographed by the sensor unit SU inspecting the subject Ob whose inspection result and image is sent to the mobile terminal device TA, and further another operation of inputting a fact or the like of a response to the subject Ob whose inspection result and image is sent to the mobile terminal device TA. The TA display part 46 is connected to the TA control processing section 41, and serves as a display device, such as an LCD and an organic EL display, for displaying contents of the predetermined operation input from the TA input part 45 and the observational information about the subject Ob (for example, a state or an image of a determined subject) observed by the subject observation system MS. Besides, in the embodiment, the TA input part 45 and the TA display part 46 constitute a touch panel. In this case, the TA input part 45 is a positional input device which effects an input by detecting an operated position in a resistive membrane way or electrostatic capacity way, for example. The touch panel provides the positional input device over the display screen of the TA display part 46. The TA display part 46 displays one or more inputtable candidate contents. For example, when a user (observer) such as a nurse or a caregiver touches a position displaying an input content which the user wants to input, the positional input device detects the touched position, and the content displayed at the detected position is input to the mobile terminal device TA as an input content operated by the user.

The TAIF section 47 is connected to the TA control processing section 41, and serves as a circuit for inputting and outputting data with an external device in accordance with a control of the TA control processing section 41, i.e., an interface circuit adopting the Bluetooth (Registered Trademark) standard, the IrDA (Infrared Data Association) standard used for infrared communication, or the USB (Universal Serial Bus) standard, or the like.

Like the SV communication IF section 13, the TA communication IF section 43 is connected to the TA control processing section 41, and serves as a circuit for performing a communication in accordance with a control of the TA control processing section 41. The TA communication IF section 43 generates a communication signal containing the data input from the TA control processing section 41 for transfer in accordance with a communication protocol used in the network NW of the subject observation system MS, and sends the generated communication signal to the other devices SU, SV, SP via the network NW. The TA communication IF section 43 receives a communication signal from the devices SU, SV, SP via the network NW, takes data from the received communication signal, converts the taken data to another one having a different format that can be processed by the TA control processing section 41, and outputs the converted data to the TA control processing section 41.

Like the SV storage section 12, the TA storage section 42 is connected to the TA control processing section 41, and serves as a circuit for storing various predetermined programs and data in accordance with a control of the TA control processing section 41. The various predetermined programs include control processing programs such as an observational processing program for processing a communication address (such as an IP address) of the administration server SV and the observational information about the observation of the subject Ob. The various predetermined data contain respective data of the observational information about the observation of the subject Ob. The TA storage section 42 includes, for example, a ROM and an EEPROM. The TA storage section 42 further includes a RAM or the like serving as a working memory of the TA control processing section 41 to store data generated during execution of the predetermined programs. The TA storage section 42 is operably provided with a terminal observational information storage part (TA observational information storage part) 421 and a terminal setting information storage part (TA sensor information storage part) 422.

The TA observational information storage part 421 stores the observational information about the observation of the subject Ob. Like the SV observational information storage part 121, the TA observational information storage part 421 stores the observational information by registering the observational information in a terminal observational information table (TA observational information table) MT-TA having the same configuration as the SV observational information table MT-SV described above with reference to FIG. 3 except for the recovery field 1217. Moreover, in FIG. 3, the reference numerals in parentheses denote respective fields of the TA observational information table MT-TA next to the reference numerals denoting the corresponding fields of the SV observational information table MT-SV. In other words, the TA observational information table MT-TA includes a sensor ID field 4211, a determination result field 4212, a determination time field 4213, a still image field 4214, a video image field 4215, and a state confirming field 4216, and further has a record per sensor ID.

The TA sensor information storage part 422 stores the sensor information about the sensor unit SU. The TA sensor information storage part 422 stores the sensor information by registering the sensor information in a terminal sensor information table (TA sensor information table) PT-TA having the same configuration as the SV sensor information table PT-SV described above with reference to FIG. 6. Moreover, in FIG. 6, the reference numerals in parentheses denote respective fields of the TA sensor information table PT-TA next to the reference numerals denoting the corresponding fields of the SV sensor information table PT-SV. The TA sensor information table PT-TA may be incorporated into the TA observational information table MT-TA. In this case, the TA observational information table MT-TA further includes an arrangement location field 4222 and a subject field 4223.

The control processing section 41 serves as a circuit for controlling respective parts of the terminal device TA in accordance with their functions, and processing the observational information about the observation of the subject Ob. Like the SV control processing section 11, the TA control processing section 41 includes, for example, a CPU and periphery circuits therearound. The TA control processing section 41 is operably provided with a terminal control part (TA control part) 411 and a terminal observation processing part (TA observation processing part) 412 owing to execution of the control processing programs.

The TA control part 411 controls the respective parts of the mobile terminal device TA in accordance with their functions to thereby control the entirety of the mobile terminal device TA.

The TA observation processing part 412 processes the observational information about the observation of the subject Ob. Specifically, when the TA communication IF section 43 receives the observational information communication signal, the TA observation processing part 412 causes the TA observational information storage part 421 and the TA sensor information storage 422 to respectively store the observational information of the subject Ob and the sensor information contained in the received observational communication signal by registering the observational information and the sensor information in the TA observational information table MT-TA and the TA sensor information table PT-TA. The TA observation processing part 412 processes an operation about display of the observational information. For example, when the TA communication IF section 43 receives an observational information signal, the TA observation processing part 412 causes the TA display part 46 to display an observational information screen image 62 providing the observational information of the subject Ob contained in the received observational information signal. When the TA communication IF section 43 receives the second acceptance notification signal functioning as the acceptance prohibition signal and the response display communication signal, the TA observation processing part 412 prohibits accepting the predetermined operation designated by the second acceptance notification signal, and causes the TA display part 46 to display a message stating that the mobile terminal device TA referred to by the received second acceptance notification signal is responding.

The mobile terminal device TA may be made up by a portable communication terminal device such as a tablet computer, a smart phone and a mobile phone.

Next, operations performed in the embodiment will be described. A subject observation system MS having the above-described configuration includes respective devices SU, SV, SP, TA which initialize and then activate necessary parts thereof when the power is turned on. Also, an administration server SV includes an SV control processing section 11 operably provided with an SV control part 111, an SV observation processing part 112, and a coordinating processing part 113 owing to execution of control processing programs thereof. A mobile terminal device TA includes a TA control processing section 41 operably provided with a TA control part 411 and a TA observation processing part 412 owing to execution of control processing programs thereof.

Briefly, the subject observation system MS having the above-described configuration observes each subject Ob by the following operations. A sensor unit SU takes outputs from a motion sensor and from an image sensor at a predetermined sampling cycle, inspects a state (condition) of the subject Ob on the basis of the taken outputs respectively from the motion sensor and the image sensor, and, when determining that the subject Ob is in a predetermined state (such as waking-up movement, leaving movement from a bed, falling down or over, and abnormally irregular motion in the embodiment) as a result of the inspection, sends to the administration server SV via a network NW a communication signal (observational information communication signal) containing: a sensor ID of the sensor unit SU; determination result information representing a result of the determination about the inspected state of the subject Ob; determination time information representing a time of the determination about the state; and image data of a still image (or one of images used for the determination, for example, the last image) of the subject Ob used for the determination. The sensor unit SU can determine the state (condition) of the subject Ob by a well-known technology. For example, the sensor unit SU causes the body motion sensor to inspect a chest motion (i.e., shrinking and bulging motion of a chest) of the subject who is breathing, and determines the chest motion as an abnormally irregular motion when inspecting a disorder in the cycle of the chest motion or an amplitude indicating a predetermined threshold or less in the chest motion. Besides, for example, the sensor unit SU causes the image sensor to acquire an image of the subject Ob, specifies a person region of the subject Ob from the acquired image, determines a posture (such as standing, sitting and lying) of the subject Ob from an aspect ratio of the specified person region, detects a position in the specified person region, and distinctively judges the waking up movement, the leaving movement from the bed, and the falling down or over in connection with the detected posture and position of the subject Ob.

Upon receipt of the observational information communication signal from the sensor unit SU via the network NW, the administration server SV causes the SV observation processing part 112 to register the sensor ID, the determination result information, the determination time information, and a communication address for obtaining the image data of the still image and the video image respectively contained in the observational information communication signal in an SV observational information table MT-SV, thereby causing the SV observational information storage part 121 to store (record) these registered information and address in association with one another. It is possible to acquire the communication address for obtaining the video image as a communication address of a sending source contained in a header of the observational information communication signal. However, this acquirement process can be omitted if a table representing an associative relationship between the sensor unit (sensor ID) and a communication address of the sensor unit SU is prepared and stored in the SV storage section 12 in advance, in addition to the SV observational information table MT-SV. During registration of the respective information in the SV observational information table MT-SV, the administration server SV causes the SV observation processing part 112 to register a flag "0" representing a default in a state confirming field 1216 and a recovery field 1217, respectively. Further, the administration server SV causes the SV observation processing part 112 to search a record that the sensor ID contained in the observational information communication signal is registered in a sensor ID field 531 from a SV sensor information table PT-SV, acquire a name of a subject Ob registered in a subject field 533 of the searched record, search a record that the acquired name of the subject Ob is registered in a subject field 541 from a subject information table DT, acquire a group ID registered in a first destination field 542 of the searched record, search a record that the acquired group ID is registered in a group field 511 from a group information table GT, acquire a name of an observer registered in an observer field 512 of the searched record, search a record that the acquired name of the observer is registered in an observer field 521 from a terminal information table TT, and acquire a terminal device ID registered in a terminal ID field of the searched record. Then, the observational information communication signal is sent to the mobile terminal device TA having the acquired terminal ID, the observational information communication signal containing the sensor ID, the determination result information, the determination time information, the still image of the subject Ob, and further an arrangement location of the sensor unit SU having the sensor ID and the name of the subject Ob being inspected by the sensor. In this manner, the state (condition) of the subject Ob is notified to a stationary terminal device SP and the mobile terminal device TA carried by the observer who is responsible for the subject Ob.

Upon receipt of the observational information communication signal from the administration server SV via the network NW, the stationary terminal device SP and the mobile terminal device TA display observational information about observation of the subject Ob contained in the observational information communication signal. An operation of the mobile terminal device TA for displaying the observational information will be described in detail below. Briefly, the subject observation system MS inspects the subject Ob through the operation by the sensor unit SU, the administration server SV, the stationary terminal device SP and the mobile terminal device TA to thereby accomplish observation of the subject Ob.

Hereinafter, an operation for displaying the observational information about the observation of the subject Ob and relevant operations in the subject observation system MS will be described. For example, operations of the mobile terminal device TA are described for explanation here. However, the stationary terminal device SP is appreciated to perform the same operations. This configuration can achieve coordination between the stationary terminal device SP and the mobile terminal device TA as well.

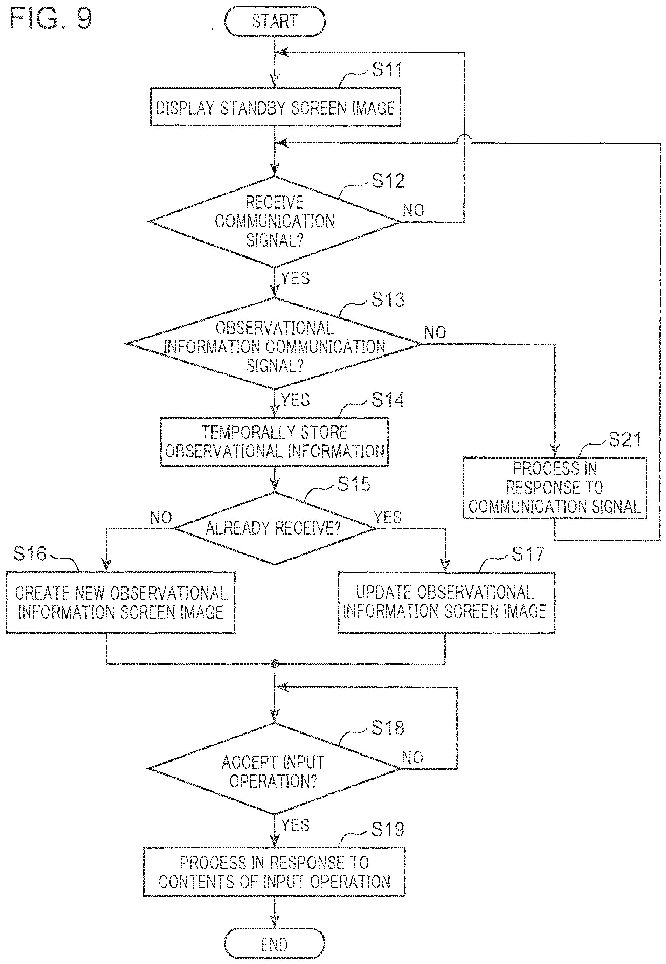

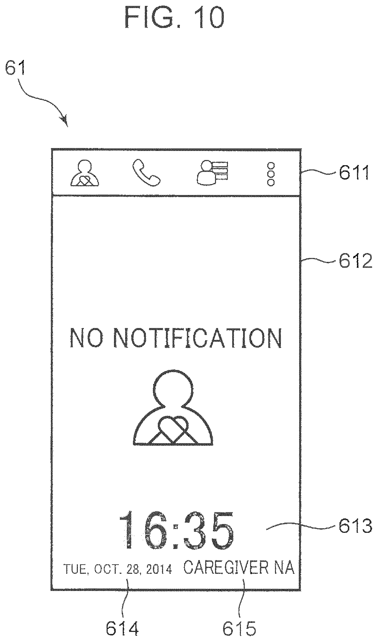

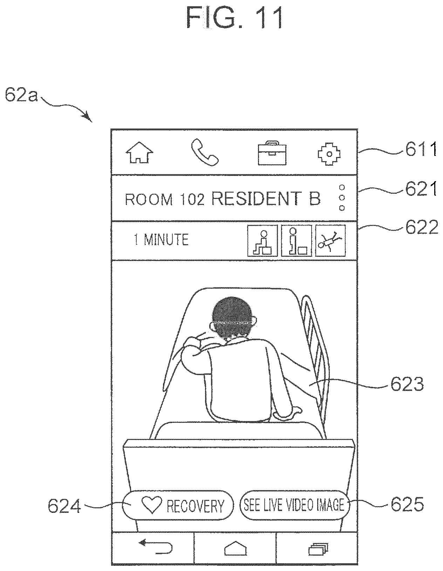

FIG. 9 is a flowchart showing an operation of the mobile terminal device for receiving notification of observational information. FIG. 10 shows an exemplary standby screen image displayed on the mobile terminal device. FIG. 11 shows an exemplary observational information screen image displayed on the mobile terminal device having received a notification of waking-up movement of a subject.

In FIG 9, the mobile terminal device TA activates when the power is turned on. For example, upon receipt of a log-in operation by an observer (user) such as a nurse or a caregiver, the mobile terminal device TA causes a TA observation processing part 412 to render a TA display part 46 to display a standby screen image indicating a standby to receive a communication signal (step S11). As shown in FIG. 10, the standby screen image 61 includes, for example, a menu bar region 611 for displaying a menu bar, a standby main region 612 for displaying a message (for example, "no notification") representing the standby and an icon, a time region 613 for displaying a current time, a calendar region 614 for displaying a day of the week, a date, a month, and a year of today, and a user name region 615 for displaying a name of a user who is logging in the mobile terminal device TA.

Then, the mobile terminal device TA causes a TA control part 411 to determine whether or not a TA communication IF section 43 receives a communication signal (S12). The mobile terminal device TA returns the process to step S11 when it is determined that the communication is not received (No), or proceeds to subsequent step S13 when it is determined that the communication signal is received (Yes).

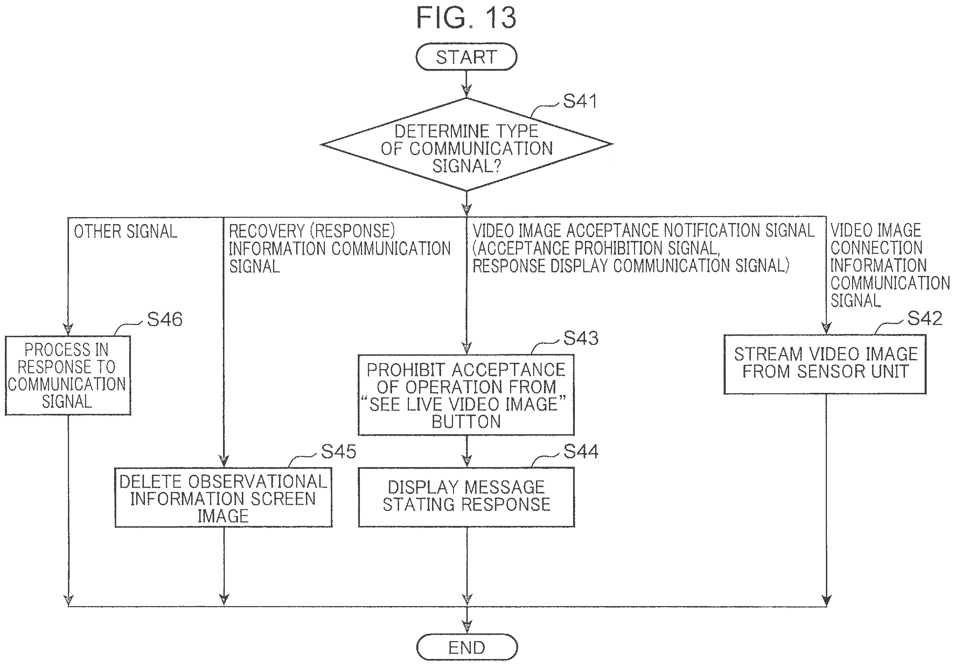

In the step S13, upon receipt of the communication signal, the mobile terminal device TA causes the TA observation processing part 412 to determine whether or not the received communication signal is an observational information communication signal. The mobile terminal device TA causes the TA control processing section 41 to execute an appropriate process in response to the received communication signal (step S21) when it is determined that the received communication is not the observational information communication signal (No), and returns the process to step S12. An exemplary appropriate process in response to the received communication signal will be described in detail later with reference to FIG. 13. To the contrary, when it is determined that the received communication signal is the observational information communication signal (Yes), the mobile terminal device TA causes the TA observation processing part 412 to register a sensor ID, determination result information, determination time information, image data of a still image, an arrangement location, and a name of a subject Ob contained in the received observational information communication signal in a TA observational information table MT-TA and a TA sensor information table PT-TA, respectively, thereby causing a TA observational information storage part 421 and a TA sensor information storage part 422 to temporarily store the registered information in association with one another, and then proceeds to step S15. During registration of the information in the TA observational information table MT-TA, the TA observation processing part 412 registers a flag "0" representing a default in a state confirming field 4216.

In the step S15, the mobile terminal device TA causes the TA observation processing part 412 to determine whether or not an observational information communication signal containing a sensor ID that is identical to the sensor ID contained in the received observational information communication signal has been previously received (in past) prior to the received observational information communication signal, and an observational information screen image for the previously received observational information communication signal has been created for display. Specifically, the TA observation processing part 412 determines whether or not a sensor ID that is identical to the sensor ID contained in the received observational information communication signal has been already registered in a sensor ID field 4211 to thereby determine whether or not the sensor ID has been previously received, and an observational information screen image for the previously received observational information communication signal has been created. When it is determined that no sensor ID that is identical to the sensor ID contained in the received observational information communication signal is registered in the sensor ID field 4211, it is judged that no observational information communication signal having the same sensor ID has been previously received (No), and then the mobile terminal device TA proceeds to step 16. To the contrary, when it is determined that the sensor that is identical to the sensor ID contained in the received observational information communication signal is registered in the sensor ID field 4211 of a record that a flag "0" is registered in the recovery field 4217, it is judged that the observational information communication signal having the same sensor ID has been previously received (Yes), and then the mobile terminal device TA proceeds to step S17.

In the step S16, the mobile terminal device TA causes the TA observation processing part 412 to create a new observational information screen image based on the respective information (data) contained in the received observational information communication signal in the TA storage section 42 to thereby render the TA display part 46 to display the created observational information screen image.

The observational information screen image is a screen image for displaying the observational information about the observation of the subject Ob. As shown in FIG. 11, an observational information screen image 62 (62a) includes, for example, a menu bar region 611, a subject name region 621 for displaying an arrangement location of a sensor unit SU having a sensor ID and a name of a subject Ob to be observed by the sensor unit SU having the sensor ID, an icon region 622 for displaying a lapse time period from a determination time (or receipt time) and an icon symbolically representing a determination result, an image region 623 for displaying an image (here, still image) photographed by the sensor unit SU having the sensor ID, a "recovery" button 624, and a "see LIVE video image" button 625. The "recovery" button 624 is used to input a fact or will of actual execution of a response (a fact or the like of a response) such as a care to the subject Ob to be observed by the sensor unit SU having the sensor ID by a user of one mobile terminal device TA, or input an instruction of notifying the stationary terminal device SP and another mobile terminal device TA that the fact or the like of the response is input, another mobile terminal device TA belonging to a first group to which the one terminal device TA belongs. The "see LIVE video image" button 625 is used to input an instruction of displaying a video image photographed by the sensor unit SU having the sensor ID.

In order to create the observational information screen image 62a based on the respective information contained in the received observational information communication signal, the TA observation processing part 412 obtains a lapse time period from a determination time contained in the received observation information, and search an icon corresponding to a determination result contained in the received observational information communication signal from the TA storage section 42 by using the determination result as a search key. It should be noted that icons respectively corresponding to determination results are stored in the TA storage section 42 in advance in connection with the determination results. Moreover, the TA observation processing part 412 creates the observational information screen image 62a by displaying the menu bar in the menu bar region 611, the arrangement location and the name of the subject Ob contained in the received observational information communication signal in the subject name region 621, the obtained lapse time period and the searched icon in the icon region 622, the image (still image) contained in the received observational information communication signal in the image region 623, and further displaying the "recovery" button 624 and the "see LIVE video image" button 625. The TA observation processing part 412 renders the TA display part 46 to display the newly created observational information screen image 62a.

Returning to FIG. 9, in the step S17, the mobile terminal device TA causes the TA observation processing part 412 to update the observational information screen image 62a on the basis of the respective information (data) contained in the received observational information communication signal to thereby render the TA display part 46 to display the updated observational information screen image.

In order to update the observational information screen image 62 on the basis of the respective information contained in the received observational information communication signal, the TA observation processing part 412 obtains a lapse time period from a determination time contained in the received observational information communication signal, and searches an icon corresponding to a determination result contained in the received observational information communication signal from the TA storage section 42 by using the determination result as a search key. Besides, the TA observation processing part 412 updates the observational information screen image 62 by displaying the obtained lapse time period and the searched icon in the icon region 622, and the image (still image) contained in the received observational communication signal in the image region 623 onto the observational information screen image 62 having been already displayed. Here, the icon region 622 includes already displayed icons corresponding to determination results contained in the previously received observational information communication signal. Thus, the currently searched icon is displayed next to the already displayed icons in a chronological order in the icon region 622. For example, when a determination result "leaving movement from the bed" of the subject Ob is notified after another determination result "waking up movement" of the subject Ob, the observational information screen image 62a where an icon representing the determination result "waking-up movement" of the subject Ob is displayed in the icon region 622 is updated by displaying the icon representing the determination result "leaving movement from the bed" of the subject Ob to the left of the icon representing the determination result "waking-up movement" in the icon region 622 on the paper. After that, the TA observation processing part 412 renders the TA display part 46 to display the updated observational information screen image 62.

Subsequent to the step S16 or S17, the mobile terminal device TA causes the TA control processing part 41 to determine whether or not a touch panel including a TA input part 45 and a TA display part 46 accepts an input operation (step S18). The mobile terminal device TA returns the process to the step S18 when it is determined that the input operation is not accepted (No), or proceeds to subsequent step S19 when it is determined that the input operation is accepted (Yes).

In the step S19, the mobile terminal device TA causes the TA control processing section 41 to perform an appropriate process corresponding to contents of the input operation, and then finishes the operation for displaying the observational information.

For example, when the mobile terminal device TA accepts an input operation of the "recovery" button 624, the TA control processing section 41 causes a TA communication IF section 43 to send to the administration server SV a communication signal (recovery notification signal, i.e., response notification signal) indicating that a user (observer) of the mobile terminal device TA has a fact or will for actual execution of a response (fact or the like of the response) such as a care to the subject Ob being displayed on the TA display part 46. The recovery notification signal contains information that the user (observer) of the mobile terminal device TA has the fact or the like of the response to the subject Ob being displayed on the TA display part 46, and contains the terminal ID and the like of the mobile terminal device TA.

Further, when the mobile terminal device TA accepts an input operation of the "see LIVE video image" button 625, the TA control processing section 41, for example, causes the TA communication IF section 43 to send to the administration server SV a communication signal (video image request communication signal) of requesting a video image of the subject Ob being displayed on the TA display part 46. The video image request communication signal contains a request for a video image, a terminal ID of a mobile terminal device TA requesting the video image, a sensor ID of a sensor unit SU generating the video image and the like. In the embodiment, the video image request communication signal functions as a first acceptance notification signal (here, video image operation acceptance notification signal) of notifying the administration server SV that a predetermined operation (here, video image request operation of requesting the video image of the subject Ob generated by a photographing part of the sensor unit SU) to the sensor unit SU is accepted by the mobile terminal device TA.

The sensor unit SU further includes a nurse call circuit for sending a nurse call to the stationary terminal device SP and the mobile terminal device TA, and a communication circuit for performing a voice communication with the stationary terminal device SP and the mobile terminal device TA, and thus may be configured to enable the nurse calling and the voice communication. In this case, the observational information screen image 62a is appreciated to further include a "talking" button 625 to input an instruction of communicatively connecting the sensor unit SU having the sensor ID with the mobile terminal device TA via the network NW. By the input operation of the "talking" button 625, the TA communication IF section 43 sends to the administration sever SV a communication signal (talking request communication signal) of requesting a voice communication with the subject Ob being displayed on the TA display part 46 and inspected by the sensor unit SU via the network NW. Upon receipt of the talking request communication signal, the administration server SV communicatively connects the mobile terminal device TA with the sensor unit SU via the network NW. Further, the talking request communication signal may function as a first acceptance notification signal (here, voice communication operation acceptance notification signal) of notifying the administration server SV that a predetermined operation (here, communication request operation of requesting a voice communication using the communication circuit of the sensor unit SU) to the sensor unit SU is accepted by the mobile terminal device TA.

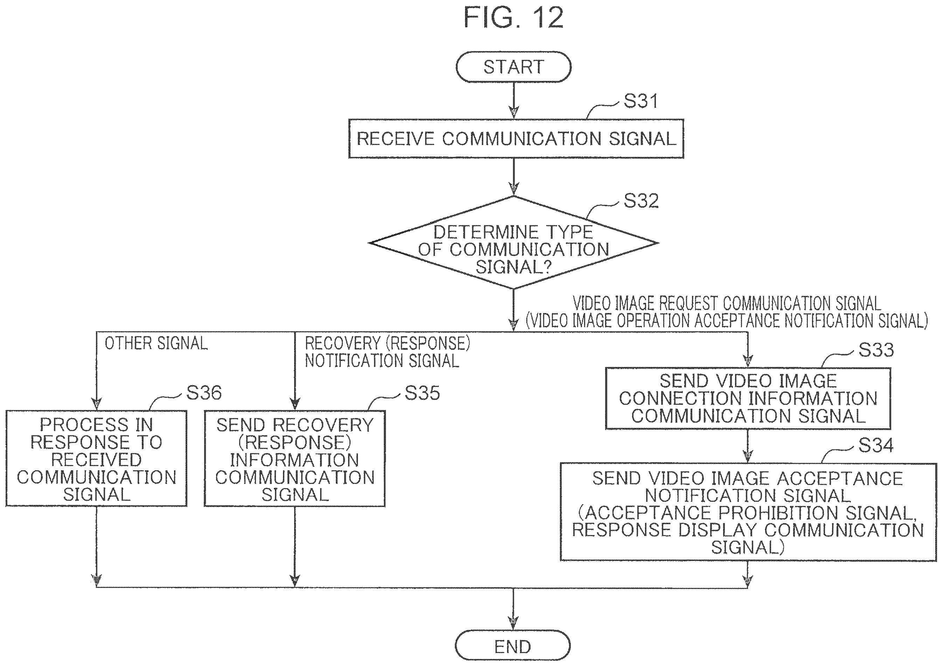

Hereinafter, described is an operation of the administration server SV for receiving the video image request communication signal (video image operation acceptance notification signal) and the recovery notification signal to be sent from the mobile terminal device TA to the administration server SV, as described above. FIG. 12 is a flowchart showing an operation of the administraton server for coordinating mobile terminal devices.

In FIG 12, when the SV communication IF section 13 receives a communication signal (step S31), the administration server SV causes a SV observation processing part 112 to determine a type of the received communication signal (step S32). The administration sever SV proceeds to step S33 when it is determined that the received communication signal is a video image request communication signal, proceeds to step S35 when it is determined that the received communication signal is a recovery notification signal, or proceeds to step S36 when the received communication signal is neither the video image request communication signal nor the recovery notification signal, that is, other signal.

In the step S33, first, in order to cause the SV observational information storage part 121 to store information as to which observer is confirming a state of a subject Ob through a video image, the administration server SV causes the SV observation processing part 112 to take out a terminal ID and a sensor ID contained in the video image request communication signal received in the step S31 respectively as information representing the mobile terminal device TA which (who) is confirming the state of the subject Ob, and information representing the sensor unit SU (whom) used for the confirmation of the state of the subject Ob. Next, the administration server SV causes the SV observation processing part 112 to search a record which registers the taken sensor ID in a sensor ID field 1211 and a flag "0" in a state confirming field 1216 and a recovery field 1217 respectively, and register the taken terminal ID in the state confirming field 1216 of the searched record to thereby perform updating. In this way, an incident that a user (observer) of the mobile terminal device TA having the terminal ID is confirming the state of the subject Ob being inspected by the sensor unit SU having the sensor ID through the video image is registered in an SV observational information table MT-SV, and the incident is stored in the SV observational information storage part 121. Then, the administration server SV causes the SV communication IF section 13 to send a communication signal (video image connection information communication signal) to the mobile terminal device TA that is an acquisition destination for a live video image, the communication signal containing a communication address (such as an IP address) of the sensor unit SU having the sensor ID contained in the video image request communication signal received in the step S31, and the mobile terminal device TA being the sending source of the video request communication signal. Specifically, the administration server SV causes the SV observation processing part 112 to search a record which registers the sensor ID contained in the video image request communication signal received in the step S31 in the sensor ID field 1211 and a flag "0" in the recovery field 1217, and acquire a communication address registered in the video image field 1215 of the searched record to thereby render the SV communication IF section 13 to send a video image connection information communication signal containing the acquired communication address and the sensor ID to the mobile terminal device TA that is the sending source of the video image request communication signal.

Subsequently, in order to avoid confirmation of the state of the subject Ob through the video image by another mobile terminal device TA in addition to one mobile terminal device TA due to notification of the incident to the mobile terminal devices TA and the stationary terminal device SP, the administrations server SV causes a coordinating processing part 113 to render the SV communication IF section 13 to send to another mobile terminal device (second mobile terminal device) TA a second acceptance notification signal (here, video image acceptance notification signal) indicating that a predetermined operation (here, video image request operation) is accepted by the one mobile terminal device (first mobile terminal device) TA having the terminal ID, the second terminal device belonging to the first group to which the first terminal device belongs and other than the first terminal device (step S34), and then finishes the operation for receiving the video image notification signal. As described above, the second acceptance notification signal (here, video image acceptance notification signal) functions as an acceptance prohibition signal (here, video image acceptance prohibition signal indicating that the second mobile terminal device TA is prohibited from accepting the video image request operation), and further functions as a response display communication signal. Specifically, the administration server SV causes the coordinating processing part 113 to search a record that a terminal device ID contained in the video image request communication signal received in the step S31 is registered in a terminal ID field 522 from a terminal information table TT, acquire a name of an observer registered in an observer field 521 of the searched record, search a record that the acquired name of the observer is registered in an observer field 512 from a group information table GT, acquire a name of another observer registered in the observer field 512 of the searched record, search a record that the acquired name of another observer is registered in the observer field 521 from the terminal information table TT, acquire a terminal ID registered in the terminal ID field 522 of the searched record to thereby render the SV communication IF section 13 to send to the mobile terminal device TA having the sensor ID a video image acceptance notification signal containing the terminal ID and the sensor ID contained in the video image request communication signal received in the step S31, an instruction of prohibiting the observational information screen image 62 corresponding to the sensor ID from accepting the video image request operation, and an instruction of causing the observational information screen image corresponding to the sensor ID to show a message stating that the observer having the acquired name is responding.

In the step S35, first, in order to cause the SV observational information storage part 121 to store information as to which observer shows a response to a subject Ob, the administration server SV causes the SV observation processing part 112 to take out a terminal ID and a sensor ID contained in the recovery notification signal received in the step S31 respectively as information representing an observer (who) of a mobile terminal device TA having accepted the fact or the like of the response to the subject Ob and information representing the subject Ob being inspected by the sensor unit SU and subjected to the response by the observer. Then, the administration sever SV causes the SV observation processing part 112 to search a record which registers the taken sensor ID in the sensor ID field 1211 and a flag "0" in the recovery field 1217, and register a flag "1" in the recovery field 1217 of the searched record to thereby perform updating. In this manner, an incident that a user (observer) of the mobile terminal device TA having the terminal ID shows the fact or the like of the response to the subject Ob being inspected by the sensor unit SU having the sensor ID is registered in the SV observational information table MT-SV, and the incident is stored in the SV observational information storage part 121. Moreover, in order to avoid showing of the fact or the like of the response to the subject Ob by another mobile terminal device TA in addition to one mobile terminal device TA due to notification of the incident to the stationary terminal device SP and the mobile terminal devices TA, the administration server SV causes the coordinating processing part 113 to render the SV communication IF section 13 to send to another mobile terminal device (second mobile terminal device) TA a recovery information communication signal indicating that an actual response to the subject is unnecessary, the second mobile terminal device TA belonging to the first group to which the one mobile terminal device (first mobile terminal device) TA having the sensor ID contained in the recovery notification signal belongs and other than the first terminal device. The recovery information communication signal contains information (recovery information) representing the unnecessity of the actual response to the subject Ob, and the sensor ID taken out from the recovery notification signal as information representing the subject Ob (whose) being inspected by the sensor unit SU but no longer requiring the response. Specifically, the administration server SV causes the coordinating processing part 113 to search a record that a terminal ID contained in the recovery notification signal received in the step S31 is registered in the terminal ID field 522 from the terminal information table TT, acquire a name of an observer registered in the observer field 521 of the searched record, search a record that the acquired name of the observer is registered in the observer field 512 from the group information table GT, acquire a name of another observer registered in the observer field 512 of the searched record, search a record that the acquired name of another observer is registered in the observer field 521 from the terminal information table TT, and acquire a terminal ID registered in the terminal ID field 522 of the searched record, to thereby render the SV communication IF section 13 to send to another mobile terminal device (i.e., the second terminal mobile device) TA having the acquired terminal ID a recovery information communication signal containing the sensor ID contained in the recovery notification signal received in the step S31 and indicating that the actual response to the subject Ob being inspected by the sensor unit SU having the sensor ID is unnecessary.

In the step 36, the administration server SV causes the SV observation processing part 112 to execute an appropriate process in response to each received communication signal, and finishes the operation for receiving the respective communication signals.

Next, described is an operation of the mobile terminal device TA having received a video image connection information communication signal, a video image acceptance notification signal (video image acceptance prohibition signal, response display communication signal) and a recovery notification signal (response notification signal) sent from the administration server SV. This operation is executed as a part of the process in the step S21 described with reference to FIG. 9. FIG. 13 is a flowchart showing an operation of the mobile terminal device for following the coordination with other mobile terminal devices.

In FIG 13, a mobile terminal device TA causes a TA observation processing part 412 to determine a type of the received communication signal (S41). The mobile terminal device TA proceeds to step S42 when it is determined that the received communication signal is a video image connection information communication signal, proceeds to step S43 when it is determined that the received communication signal is a video image acceptance notification signal (video image acceptance prohibition signal, response display communication signal), proceeds to step S45 when it is determined that the received communication signal is a recovery information communication signal, or proceeds to step S46 when it is determined that the communication signal is none of the video image connection information communication signal, the video image acceptance notification signal, and the recovery information communication signal, that is, other signal.

In the step S42, the mobile terminal device TA causes the TA observation processing part 412 to take out a communication address of a sensor unit SU contained in the video image connection information communication signal received in the step S12, connect the sensor unit SU thereto via a network NW by using the taken communication address of the sensor unit SU, download a video image from the sensor unit SU, and display the video image of the subject Ob in an image region 623 of an observational information screen image 62a by means of, for example, streaming reproduction. The mobile terminal device TA further causes the TA observation processing part 412 to search a record that a sensor ID contained in the received video connection information communication signal is registered in a sensor ID field 4211 from a TA observational information table MT-TA, registers a flag "1" in a state confirming field 4216 of the searched record, and finishes the operation for receiving the video image connection information communication signal.

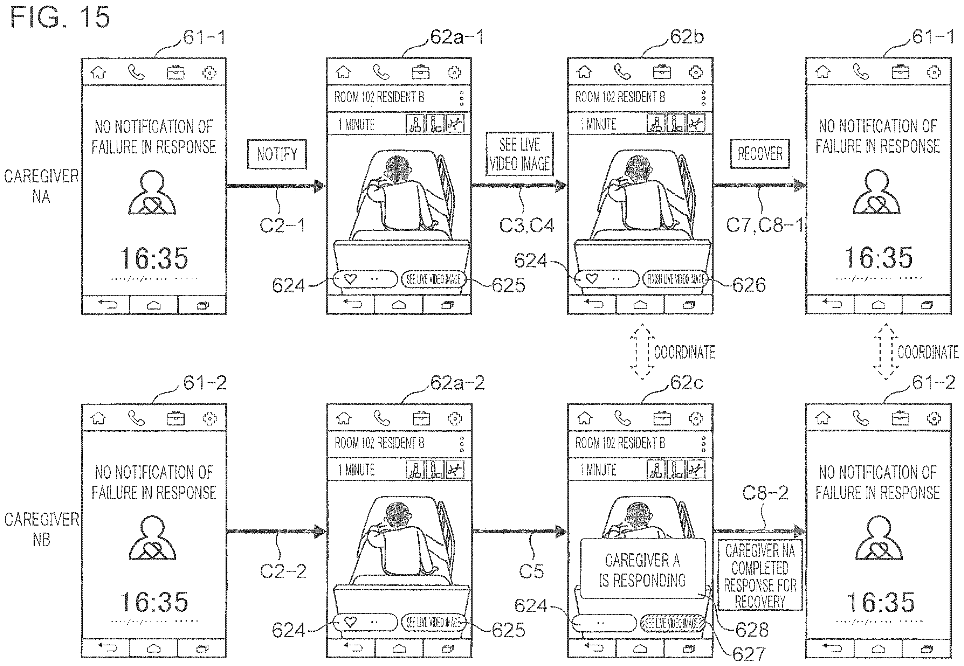

In the step S43, in order to cause the TA observational information storage part 421 to store information as to which observer is confirming a state of a subject Ob, the mobile terminal device TA causes the TA observation processing part 412 to take out a terminal ID and a sensor ID contained in the video image acceptance notification signal received in the step S12 respectively as information representing one mobile terminal device TA which is confirming the state of the subject Ob, and information representing a sensor unit SU used for confirming the state of the subject Ob. Moreover, the mobile terminal device TA causes the TA observation processing part 412 to search a record that the taken sensor ID is registered in the sensor ID field 4211, and register the taken terminal ID in the state confirming field 4216 of the searched record to thereby perform updating. In this manner, an incident that a user (observer) of the mobile terminal device TA having the terminal ID is confirming the state of the subject Ob being inspected by the sensor unit SU having the sensor ID is registered in the TA observational information table MT-TA, and the incident is stored in the TA observational information storage part 421. Further, in order to avoid confirmation of the state of the subject Ob by a mobile terminal device TA, the mobile terminal device TA causes the TA observation processing part 412 to prohibit the observational information screen image 62 from accepting the video image request operation, the observational information screen image 62 corresponding to the sensor ID contained in the video image acceptance notification signal received in the step S12. Specifically, the mobile terminal device TA causes the TA observation processing part 412 to change the display color of the "see LIVE video image" button 625 to gray on an observational information screen image 62a (62a-2) corresponding to the sensor ID, for example, as shown in FIG 15 to be described later. In this manner, the TA display part 46 displays the observational information screen 62c having a grayed "see LIVE video image" button 627. Thus, even when the user (observer) performs an input operation by the "see LIVE video image" button 627, the mobile terminal device TA causes the TA observation processing part 412 not to accept the input operation, thereby disabling the input operation.

Subsequently, the mobile terminal device TA causes the TA observation processing part 412 to display information that another mobile terminal device TA is confirming the state of the subject Ob (S44), and finishes the operation for receiving the video image acceptance notification signal. Specifically, as shown in FIG. 15 to be described later, the mobile terminal device TA causes the TA observation processing part 412, for example, to display a message stating "caregiver NA is responding" 628 on an observational information screen image 62a corresponding to the sensor ID contained in the video image acceptance notification signal received in the step S12. This configuration allows the TA display part 46 to display an observational information screen image 62c having the message stating "caregiver NA is responding" 628.

In the step S45, in order to cause the TA observational information storage part 421 to store information as to which subject Ob no longer requires the response, the mobile terminal device TA causes the TA observation processing part 412 to take out a sensor ID contained in the recovery information communication signal received in the step S12. Next, the mobile terminal device TA causes the TA observation processing part 412 to search a record which registers the taken sensor ID in the sensor ID field 4211 from the TA observational information table MT-TA, and delete the searched record. Then, the mobile terminal device TA causes the TA observation processing part 412 to delete (eliminate) an observational information screen image 62 in connection with the taken sensor ID from the TA storage section 42, and finishes the operation for receiving the recovery information communication signal.

In the step S46, the mobile terminal device TA causes the TA observation processing part 412 to execute an appropriate process in response to the received communication signal, and finishes the operation for receiving the communication signal.

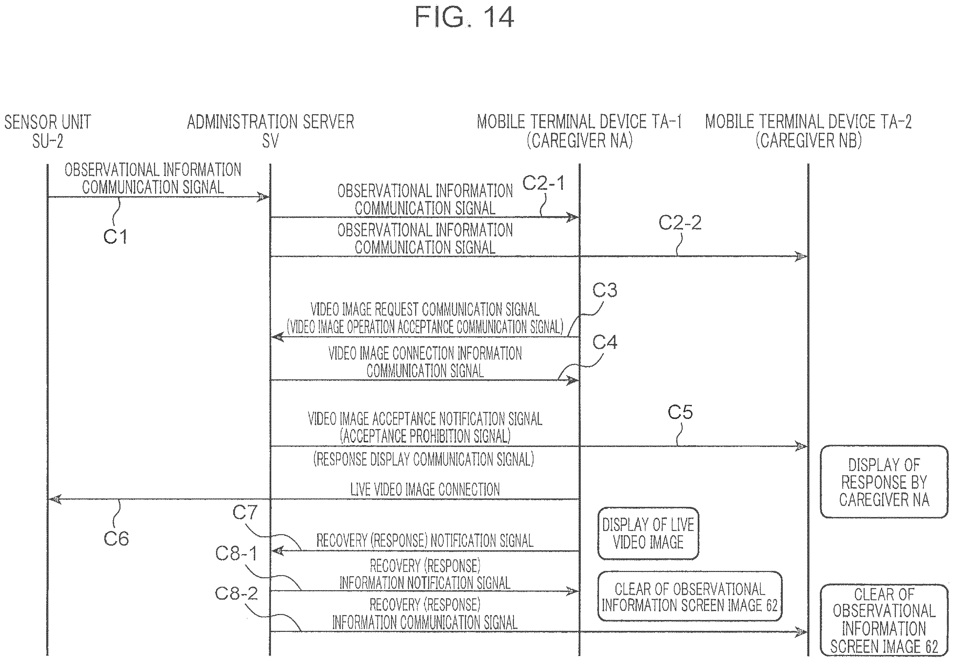

Subsequently, an exemplary coordination between mobile terminal devices will be described. FIG. 14 shows a sequence diagram illustrating an exemplary coordination between two mobile terminal devices. FIG. 15 shows a diagram illustrating screen image shifts in connection with an exemplary coordination between two mobile terminal devices.

In the embodiment, as shown in FIGS. 1, 14 and 15, a coordination between a mobile terminal device TA-1 used by a caregiver NA and a mobile terminal device TA-2 used by a caregiver NB among a plurality of mobile terminal devices TA will be described. In the embodiment, the mobile terminal devices TA-1, TA-2 belong to a same group. Also, observational information includes only information about a sensor unit SU-2, and does not include any other information at all. Mobile terminal IDs of the mobile terminal devices TA-1, TA-2 are respectively denoted by TA-1, TA-2. Further, a sensor ID of the sensor unit SU-2 inspecting a subject Ob-2 (resident B) is denoted by SU-2.

In FIGS. 14 and 15, when the caregiver NA logs in the mobile terminal device TA-1, the mobile terminal device TA-1 causes a TA display part 46-1 to display a standby screen image 61-1 in the step S11. Similarly, when the caregiver NB logs in the mobile terminal device TA-2, the mobile terminal device TA-2 causes a TA display part 46-2 to display a standby screen image 61-2 in the step S11. It should be noted that, when it is necessary to distinguish the configuration of the mobile terminal device TA-1 from that of the mobile terminal device TA-2, respective parts in the configuration of the mobile terminal device TA-1 are denoted with a subscript "-1" and those in the configuration of the mobile terminal device TA-2 are denoted with a subscript "-2" for explanation.

The sensor unit SU-2 inspecting the subject Ob-2 (resident B) determines a state (condition) of the subject Ob-2 by the above-described operations. When an observational information communication signal is sent (C1), each of the mobile terminal devices TA-1, TA-2 receives the observational information signal via the administration server SV (C2-1, C2-2).

Upon receipt of the observational information communication signal, the mobile terminal device TA-1 causes the TA display part 46-1 to display an observational information screen image 62a-1 about the subject Ob-2 (the sensor unit SU-2 having the sensor ID denoted by SU-2) in the steps S12 through S16. Similarly, the mobile terminal device TA-2 causes the TA display part 46-2 to display an observational information screen image 62a-2 about the subject Ob-2 in the steps S12 through S16.

In this case, when the caregiver NA performs an input operation by the "see LIVE video image" button 625 of the mobile terminal device TA-1, the mobile terminal device TA-1 sends a video image request notification signal (video image operation acceptance notification signal) to the administration server SV (C3) in the steps S18 and S19. Upon receipt of the video image request communication signal, the administration server SV sends a video image connection information communication signal to the mobile terminal device TA-1 (C4) as a response in the step S31 through S33, and a video image acceptance notification signal (video image acceptance prohibition signal, response display communication signal) to the mobile terminal device TA-2 (C5).

Upon receipt of the video image connection information communication signal, the mobile terminal device TA-1 is connected to the sensor unit SU-2 via the network NW in the step S42, and displays a video image of the subject Ob-2 in an image region 623 of the observational information screen image 62b by means of streaming reproduction of the video image from the sensor unit SU (C6) as shown in FIG. 15. Displayed on the observational information screen image 62b is a "finish LIVE video image" button 626 in place of the "see LIVE video image" button 625. The "finish LIVE video image" button 626 is used to input an instruction of finishing the video image photographed by the sensor unit SU-2.

In contrast, upon receipt of the video image acceptance notification signal, the mobile terminal device TA-2 changes the display color of the "see LIVE video image" button 625 to gray on the observational information screen image 62a-2, and displays a message stating "caregiver NA is responding" 628 in steps S43 and S44, as shown in FIG 15. This configuration allows the TA display part 46-2 to display an observational information screen image 62c including a grayed "see LIVE video image" button 627, and a message stating "caregiver NA is responding" 628, to thereby disable the user (observer) to perform the input operation by the "see LIVE video image" button 627. Accordingly, the mobile terminal devices TA-1, TA-2 are coordinated with each other.

In this configuration, when the caregiver NA performs an input operation by a "recovery" button 624 at the mobile terminal device TA-1, the mobile terminal device TA-1 sends a recovery notification signal to the administration server SV in the steps S18 and S19 (C7).

Upon receipt of the recovery notification signal, the administration server SV sends a recovery information communication signal to the mobile terminal devices TA-1, TA-2 in the steps S31, 32 and S35 (C8-1, C8-2).

Upon receipt of the recovery information communication signal, the mobile terminal devices TA-1, TA-2 delete the observational information of the sensor unit SU-2 from the TA observational information table MT-TA in the step S45, delete the observational information screen images 62a-1, 62a-2 for the sensor unit SU-2, and display standby screen images 61-1, 61-2 respectively in the step S45. Accordingly, the mobile terminal devices TA-1, TA-2 are coordinated with each other.

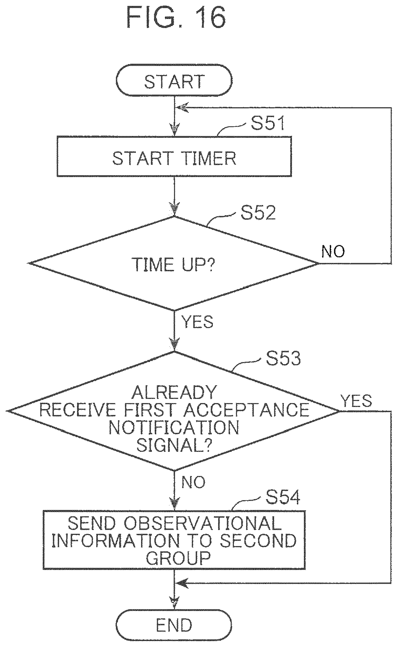

Hereinafter, described is an operation for sending an observational information communication signal to a mobile terminal device belonging to a second group when a first acceptance notification signal (video image request communication signal, i.e. video image operation acceptance notification signal, in the embodiment) is not received within a predetermined time period after the observational information communication signal is sent to a mobile terminal device belonging to a first group. FIG. 16 is a flowchart showing an operation of the administration server for sending observational information to a second group.

In FIG. 16, as described above, upon receipt of the observational information communication signal from the sensor unit SU, the administration server SV causes the SV observation processing part 112 to perform a necessary process to thereby render the SV communication IF section 13 to send the observational information communication signal to the mobile terminal device TA of the first group (C2-1, C2-2 in FIG. 14). In this case, the coordinating processing part 113 generates a timer to count the predetermined time period (for example, five, ten or twenty minutes, or the like) in association with a sensor ID contained in the observational information communication signal received from the sensor unit SU, and start the timer relevant to the sensor ID (step S51).

After that, the coordinating processing part 113 determines whether or not the timer times up (step S52). When the timer does not time up (No), the coordinating processing part 113 returns the process to the step S51. To the contrary, when the timer times up (Yes), the coordinating processing part 113 determines whether or not a first acceptance notification signal (video image request communication signal, i.e., video image operation acceptance notification signal, in the embodiment) corresponding to the sensor ID of the timer has been previously received (step S53). Specifically, the coordinating processing part 113 search a record which registers the sensor ID of the timer in a sensor ID field 1211 and a flag "0" in a recovery field 1217 from the SV observational information table MT-SV. When the record is not found as a result of the search, this means that recovery has been already performed. Thus, the coordinating processing part 113 determines that the first acceptance notification signal has been previously received regardless of receipt or non-receipt of the first acceptance notification signal, and finishes this process. When the record is found as a result of the search, the coordinating processing part 113 judges contents registered in a state confirming field 1216 of the searched record. When a terminal ID is registered in the state confirming field 1216 as a result of the judgment, the coordinating processing part 113 determines that the first acceptance notification signal has been previously received, and finishes this process. In contrast, when a flag "0" is registered in the state confirming field 1216, the coordinating processing part 113 determines that the first acceptance notification signal has not been received, and proceeds to a subsequent step S54.

In the step S54, the coordinating processing part 113 renders the SV communication IF section 13 to send to a mobile terminal device TA of the second group an observational information communication signal in connection with the sensor ID of the timer, and finishes this process. Specifically, the coordinating processing part 113 searches a record that the sensor ID of the timer is registered in a sensor ID field 531 from the SV sensor information table PT-SV, acquires a name of a subject Ob registered in a subject field 533 of the searched record, searches a record that the acquired name of the subject Ob is registered in a subject field 541 from the subject information table DT, acquires a group ID registered in a second destination field 543 of the searched record, searches a record that the acquired group ID is registered in a group field 511 from the group information table GT, acquires a name of an observer registered in an observer field 512 of the searched record, searches a record that the acquired name of the observer is registered in an observer field 521 from the terminal information table TT, and acquires a terminal ID registered in a terminal ID field of the searched record. Then, the observational information communication signal is sent to the mobile terminal device TA having the acquired terminal ID. In this manner, the state (condition) of the subject Ob is notified to the mobile terminal device TA of the second group.

As described above, a subject observation system MS, and an administration server SV and a method for use in the system in the embodiment can achieve coordination between the mobile terminal devices TA by causing the coordinating processing part 113, upon receipt of the first acceptance notification signal (video image request communication signal, i.e., video image operation acceptance notification signal, in the embodiment) from one mobile terminal device (first mobile terminal device) TA, to send a second acceptance notification signal to another mobile terminal device (second mobile terminal device) TA belonging to a first group to which the first terminal device belongs and other than the first mobile terminal device TA. A second user (second observer) using the second mobile terminal device TA having received the second acceptance notification signal can recognize that a first user (first observer) using the first mobile terminal device TA expresses a will of a response to the notified observational information by seeing that a predetermined operation is accepted by the first mobile terminal device TA.

A subject observation system MS, and an administration server SV and a method for use in the system can achieve coordination between the mobile terminal devices TA and avoid acceptance of the predetermined operation thereat by, when the predetermined operation (video image request operation in the embodiment) is accepted by a mobile terminal device TA of a first group, prohibiting another mobile terminal device TA of the first group from accepting the predetermined operation. When the predetermined operation is a video image request operation of requesting a video image of the subject Ob generated by the photographing part of the sensor unit SU, not all the terminal devices can freely see the subject, and thus the privacy of the subject Ob can be protected.

A subject observation system MS, and an administration sever SV and a method for use in the system in the embodiment allow a terminal device to display a video image by a video image request operation when the sensor unit SU includes a photographing part, or allow the mobile terminal device TA to perform a voice communication with the sensor unit SU when the sensor unit SU includes a sound input and output part.

A subject observation system MS, and an administration server SV and a method for use in the system in the embodiment allow the second observer using the second mobile terminal device TA to clearly recognize that the first observer using the first mobile terminal device TA is responding by causing the second mobile terminal device TA to display a message stating the response.

A subject observation system MS, and an administration server SV and a method for use in the system in the embodiment can prevent failure in response and achieve coordination between groups by sending observational information to the second group when the first group fails to respond within a predetermined time period.

Various aspects of technologies are disclosed in this specification as described above. Main technologies among them will be summarized below.

A central processing apparatus according to one aspect for use in a subject observation system for observing a subject as a watching target includes a sensor unit for inspecting the subject , the central processing apparatus communicatively connected with the sensor unit for administrating observational information about the subject in connection with a received inspection result from the sensor unit, and a plurality of terminal devices communicatively connected with the central processing apparatus to receive and display the observational information, and the central processing apparatus including: a communicator which performs a communication; a destination terminal group information storage which stores an associative relationship between the sensor unit and a first group composed of a specified number of terminal devices that are first destinations to which the observational information about the subject in connection with the received inspection result from the sensor unit is sent; and a coordinating processor which renders the communicator to send, when the communicator receives from a first terminal device a first acceptance notification signal having been sent to notify the central processing apparatus that a predetermined operation to the sensor unit is accepted by the first terminal device, to a second terminal device a second acceptance notification signal indicating that the predetermined operation is accepted by the first terminal device, the second terminal device belonging to the first group to which the first terminal device belongs and other than the first terminal device.

The central processing apparatus can achieve coordination between the terminal devices by causing the coordinating processing part to, upon receipt of the first acceptance notification signal from the first terminal device, send the second acceptance notification signal to the second mobile terminal device belonging to the first group to which the first terminal device belongs and other than the first terminal device. A second user (second observer) using the second terminal device having received the second acceptance notification signal can recognize that a first user (first observer) using the first terminal device expresses a will of a response to the notified observational information by seeing that the predetermined operation is accepted by the first terminal device.

In a central processing apparatus according to another aspect, the coordinating processor renders the communicator to further send to the second terminal device an acceptance prohibition signal indicating that the second terminal device is prohibited from accepting the predetermined operation. Preferably, the acceptance prohibition signal serves as the second acceptance notification signal.

The central processing apparatus can achieve coordination between the terminal devices and avoid acceptance of the predetermined operation thereat by, when the predetermined operation is accepted by a terminal device of the first group, prohibiting another terminal device of the first group from accepting the predetermined operation. When the predetermined operation is a video image request operation of requesting a video image of the subject generated in the photographing part of the sensor unit, not all the terminal devices can freely see the subject, and thus the privacy of the subject can be protected.

In a central processing apparatus according to further another aspect, the sensor unit includes at least one of a photographing part for generating a video image of the subject and a sound input and output part for inputting and outputting a sound, and the predetermined operation involves at least one of a video image request operation of requesting the video image of the subject generated by the photographing part of the sensor unit and a communication request operation of requesting a voice communication via the sound input and output part of the sensor unit.

The central processing apparatus allows the terminal device to display a video image by a video image request operation when the sensor unit includes a photographing part, or allows the terminal device to perform a voice communication with the sensor unit when the sensor unit includes a sound input and output part.

In a central processing apparatus according to still another aspect, the coordinating processor renders the communicator to further send to the second terminal device a response display communication signal of causing the second terminal device to display a message stating that the first terminal device is responding. Preferably, the response display communication signal serves as the acceptance prohibition signal. Preferably, the response display communication signal serves as the second acceptance notification signal.

The central processing apparatus allows a second observer using the second terminal device to clearly recognize that a first observer using the first terminal device is responding by causing the second terminal device to display a message stating the response.

In a central processing apparatus according to still further another aspect, the destination terminal group information storage further stores an associative relationship between the sensor unit and a second group composed of another specified number of terminal devices that are second destinations to which the observational information about the subject in connection with the received inspection result from the sensor unit is sent, the observational information being sent to the second group next to the first group, and the coordinating processor renders the communicator to send to the terminal devices in association with the sensor unit and belonging to the second group the observational information about the subject in connection with the received inspection result from the sensor unit, when the communicator receives no first acceptance notification signal until a predetermined time period lapses from a timing at which the communicator sends to the terminal devices in association with the sensor unit and belonging to the first group the observational information about the subject in connection with the received inspection result from the sensor unit.

The central processing apparatus can prevent failure in response and achieve coordination between the groups by sending observational information to the second group when the first group fails to respond within the predetermined time period.

A central processing method according to another aspect is for centrally processing a subject observation system for observing a subject as a watching target, the system including a sensor unit for inspecting the subject, a central processing apparatus communicatively connected with the sensor unit for administrating observational information about the subject in connection with a received inspection result from the sensor unit, and a plurality of terminal devices communicatively connected with the central processing apparatus for receiving and displaying the observational information, the central processing method including: a storing step of storing in a destination terminal group information storage an associative relationship between the sensor unit and a first group composed of a specified number of terminal devices that are first destinations to which the observational information about the subject in connection with the received inspection result from the sensor unit is sent; and a coordination processing step of sending via a communicator, when the communicator receives from a first terminal device a first acceptance notification signal having been sent to notify the central processing apparatus that a predetermined operation to the sensor unit is accepted by the first terminal device, to a second terminal device a second acceptance notification signal indicating that the predetermined operation is accepted by the first terminal device, the second terminal device belonging to the first group which the first terminal device belongs to and other than the first terminal device.

The central processing method can achieve coordination between the terminal devices in the coordination processing step by, upon receipt of the first acceptance notification signal from the first terminal device, sending the second acceptance notification signal to the second terminal device belonging to the first group to which the first terminal device belongs and other than the first terminal device. A second user (second observer) using the second terminal device having received the second acceptance notification signal can recognize that a first user (first observer) using the first terminal device expresses a will of a response to the notified observational information by seeing that the predetermined operation is accepted by the first terminal device.

A subject observation system according to further another aspect for observing a subject who is a watching target includes: a sensor unit for inspecting the subject; a central processing apparatus having one of the aforementioned configurations and communicatively connected with the sensor unit to administrate observational information about the subject in connection with a received inspection result from the sensor unit; and a plurality of terminal devices communicatively connected with the central processing apparatus for receiving and displaying the observational information.

The subject observation system can achieve coordination between the terminal devices.

Although the present invention has been fully described by way of example with reference to the accompanying drawings, it is to be understood that various changes and modifications will be apparent to those skilled in the art. Therefore, unless otherwise such changes and modifications depart from the scope of the present invention as defined by the claims, they should be construed as being included therein.

### Industrial Applicability

The present invention can provide a central processing apparatus and a central processing method for use in a subject observation system, and a subject observation system.

## Claims

1. A central processing apparatus (SV) for use in a subject observation system (MS) for observing a subject as a watching target (Ob), the system including a sensor unit (SU) for inspecting the subject, the central processing apparatus communicatively connected with the sensor unit for administrating observational information about the subject in connection with a received inspection result from the sensor unit, and a plurality of terminal devices (SP,TA) communicatively connected with the central processing apparatus to receive and display the observational information, the central processing apparatus comprising:
a communicator (13) configured to perform a communication;
a destination terminal group information storage (122) configured to store an associative relationship between the sensor unit and a first group composed of a plurality of terminal devices that are first destinations to which the observational information about the subject in connection with the received inspection result from the sensor unit is sent; and
a coordinating processor configured to cause the communicator (13) to send, when the communicator receives from a first terminal device (TA-1) a first acceptance notification signal (C3) having been sent to notify the central processing apparatus (SV) that a predetermined operation to the sensor unit (SU) is accepted by the first terminal device (TA-1), to a second terminal device (TA-2) a second acceptance notification signal (C5) indicating that the predetermined operation is accepted by the first terminal device (TA-1), the second terminal device (TA-2) belonging to the first group (Gr1) to which the first terminal device (TA-1) belongs and distinct from the first terminal device (TA-1).

2. A central processing apparatus (SV) according to claim 1, wherein
the coordinating processor is configured to cause the communicator (13) to further send to the second terminal device (TA-2) an acceptance prohibition signal (C5) indicating that the second terminal device (TA-2) is prohibited from accepting the predetermined operation.

3. A central processing apparatus (SV) according to claim 1 or 2, wherein
the sensor unit (SU) includes at least one of a photographing part for generating a video image of the subject (Ob) and a sound input and output part for inputting and outputting a sound, and
the predetermined operation involves at least one of a video image request operation of requesting the video image of the subject generated by the photographing part of the sensor unit and a communication request operation of requesting a voice communication via the sound input and output part of the sensor unit.

4. A central processing apparatus (SV) according to any one of claims 1 to 3, wherein
the coordinating processor is configured to cause the communicator to further send to the second terminal device (TA-2) a response display communication signal (C5) causing the second terminal device to display a message (628) stating that the first terminal device (TA-1) is responding.

5. A central processing apparatus (SV) according to any one of claims 1 to 4, wherein
the destination terminal group information storage (122) is further configured to store an associative relationship between the sensor unit (SU) and a second group (Gr2) composed of another specified number of terminal devices (SP,TA) that are second destinations to which the observational information about the subject in connection with the received inspection result from the sensor unit (SU) is sent, the observational information being sent to the second group (Gr2) next to the first group (Gr1), and the coordinating processor is configured to cause the communicator (13) to send to the terminal devices (TA, SP) in association with the sensor unit (SU) and belonging to the second group the observational information about the subject (Ob) in connection with the received inspection result from the sensor unit, when the communicator (13) receives no first acceptance notification signal until a predetermined time period lapses from a timing at which the communicator sends to the terminal (TA, SP) in association with the sensor unit (SU) and belonging to the first group (Gr1) the observational information about the subject (Ob) in connection with the received inspection result from the sensor unit.

6. A central processing method for centrally managing a subject observation system (MS) for observing a subject (Ob) as a watching target, the system including a sensor unit (SU) for inspecting the subject, a central processing apparatus (SV) communicatively connected with the sensor unit for administrating observational information about the subject in connection with a received inspection result from the sensor unit, and a plurality of terminal devices (SP,TA) communicatively connected with the central processing apparatus (SV) for receiving and displaying the observational information, the central processing method comprising:
a storing step of storing in a destination terminal group information storage (122) an associative relationship between the sensor unit (SU) and a first group (Gr1) composed of a plurality of terminal devices that are first destinations to which the observational information about the subject in connection with the received inspection result from the sensor unit (SU) is sent; and
a coordination processing step of sending via a communicator, when the communicator (13) receives from a first terminal device (TA-1) a first acceptance notification signal having been sent to notify the central processing apparatus (SV) that a predetermined operation to the sensor unit (SU) is accepted by the first terminal device (TA-1), to a second terminal device (TA-2) a second acceptance notification signal indicating that the predetermined operation is accepted by the first terminal device, the second terminal device belonging to the first group (Gr1) which the first terminal device (TA-1) belongs to and distinct from the first terminal device (TA-1).

7. A subject observation system (MS) for observing a subject (Ob) who is a supervised target, the system comprising:
a sensor unit (SU) for inspecting the subject;
a central processing apparatus (SV) according to any one of claims 1 to 5 communicatively connected with the sensor unit (SU) to administrate observational information about the subject (Ob) in connection with a received inspection result from the sensor unit; and
a plurality of terminal devices (SP, TA) communicatively connected with the central processing apparatus (SV) for receiving and displaying the observational information.

## Patentansprüche

1. Zentraler Prozessor (SV) zur Verwendung in einem Personenbeobachtungssystem (MS) zur Beobachtung einer Person als Beobachtungsziel (Ob),
wobei das System eine Sensoreinheit (SU) zur Überprüfung der Person aufweist, wobei der zentrale Prozessor kommunikativ mit der Sensoreinheit verbunden ist zur Verwaltung der Beobachtungsinformationen Ober die Person in Verbindung mit einem von der Sensoreinheit empfangenen Überprüfungsergebnis, und eine Vielzahl von Endgeräten (SP, TA), die kommunikativ mit dem zentralen Prozessor verbunden sind, um die Beobachtungsinformationen zu empfangen und anzuzeigen, wobei der zentrale Prozessor aufweist:
einen Kommunikator (13), der dazu ausgebildet ist, eine Kommunikation zu führen;
einen Informationsspeicher (122) für eine Bestimmungsendgerätegruppe, der dazu ausgebildet ist, eine assoziative Verknüpfung zwischen der Sensoreinheit und einer ersten Gruppe, die aus einer Vielzahl von Endgeräten besteht, zu speichern, welche erste Bestimmungsziele sind, an welche die Beobachtungsinformationen über die Person in Verbindung mit dem von der Sensoreinheit empfangenen Überprüfungsergebnis geschickt werden; und
einen Koordinationsprozessor, der dazu ausgebildet ist, den Kommunikator (13) zu veranlassen, wenn der Kommunikator von einem ersten Endgerät (TA-1) ein erstes Akzeptanzbenachrichtigungssignal (C3) empfängt, das an ein zweites Endgerät (TA-2) gesendet wurde, um den zentralen Prozessor (SV) zu benachrichtigen, dass ein vorbestimmter Vorgang an der Sensoreinheit (SU) von dem ersten Endgerät (TA-1) akzeptiert wird, ein zweites Akzeptanzbenachrichtigungssignal (C5) auszusenden, das anzeigt, dass der vorbestimmte Vorgang von dem ersten Endgerät (TA-1) akzeptiert wird, wobei das zweite Endgerät (TA-2) zu der ersten Gruppe (Gr1) gehört, zu welcher das erste Endgerät (TA-1) gehört, und sich aber von dem ersten Endgerät (TA-1) unterscheidet.

2. Zentraler Prozessor (SV) gemäß Anspruch 1, wobei
der Koordinationsprozessor dazu ausgebildet ist, den Kommunikator (13) zu veranlassen, an das zweite Endgerät (TA-2) ferner ein Akzeptanzuntersagungssignal (C5) zu senden, das anzeigt, dass es dem zweiten Endgerät (TA-2) untersagt ist, den vorbestimmten Vorgang zu akzeptieren,

3. Zentraler Prozessor (SV) gemäß Anspruch 1 oder 2, wobei
die Sensoreinheit (SU) zumindest ein Element von einem Photographie-Element zur Erzeugung eines Videobildes der Person (Ob) und einem Toneingabe und -ausgabeelement zur Eingabe und Ausgabe eines Tons aufweist, und
der vorbestimmte Vorgang zumindest einen Vorgang umfasst aus einem Anfragevorgang für ein Videobild, bei dem ein von dem Photographie-Element der Sensoreinheit erzeugtes Videobild der Person angefragt wird, und einem Kommunikations-Anfragevorgang, bei dem eine Sprachkommunikation über das Toneingabe und -ausgabeelement der Sensoreinheit angefragt wird.

4. Zentraler Prozessor (SV) gemäß irgendeinem der Ansprüche 1 bis 3, wobei
der Koordinationsprozessor dazu ausgebildet ist, den Kommunikator zu veranlassen, ferner an das zweite Endgerät (TA-2) ein Antwort-Anzeige-Kommunikationssignal (C5) zu senden, welches das zweite Endgerät dazu veranlasst, eine Nachricht (628) anzuzeigen, welche aussagt, dass das erste Endgerät (TA-1) antwortet

5. Zentraler Prozessor (SV) gemäß irgendeinem der Ansprüche 1 bis 4, wobei
der Informationsspeicher (122) für eine Bestimmungsendgerätegruppe ferner dazu ausgebildet ist, eine assoziative Verknüpfung zwischen der Sensoreinheit (SU) und einer zweiten Gruppe (Gr2), die aus einer weiteren bestimmten Anzahl von Endgeräten (SP, TA) besteht, zu speichern, welche zweite Bestimmungsziele sind, an welche die Beobachtungsinformationen über die Person in Verbindung mit dem von der Sensoreinheit (SU) empfangenen Überprüfungsergebnis geschickt werden, wobei die Beobachtungsinformationen an die zweite Gruppe (Gr2) neben der ersten Gruppe (Gr1) geschickt werden, und
der Koordinationsprozessor dazu ausgebildet ist, den Kommunikator (13) zu veranlassen, an die Endgeräte (TA, SP), die in Verbindung mit der Sensoreinheit (SU) stehen und die zu der zweiten Gruppe gehören, die Beobachtungsinformationen über die Person (Ob) in Verbindung mit dem von der Sensoreinheit empfangenen Überprüfungsergebnis zu schicken, wenn der Kommunikator (13) kein erstes Akzeptanzbenachrichtigungssignal erhält bis zum Ablauf einer vorbestimmten Zeitspanne ab dem Zeitpunkt, zu welchem der Kommunikator den Endgeräten (TA, SP), die in Verbindung mit der Sensoreinheit (SU) stehen und die zu der ersten Gruppe gehören, die Beobachtungsinformationen über die Person (Ob) in Verbindung mit dem von der Sensoreinheit empfangenen Überprüfungsergebnis schickt,

6. Zentrales Verarbeitungsverfahren zur zentralen Verwaltung eines Personenüberwachungssystems (MS) zur Beobachtung einer Person (Ob) als Beobachtungsziel (Ob), wobei das System eine Sensoreinheit (SU) aufweist zur Überprüfung der Person, einen zentralen Prozessor (SV), der kommunikativ mit der Sensoreinheit verbunden ist zur Verwaltung der Beobachtungsinformationen über die Person in Verbindung mit einem von der Sensoreinheit empfangenen Überprüfungsergebnis, und eine Vielzahl von Endgeräten (SP, TA), die kommunikativ mit dem zentralen Prozessor (SV) verbunden sind, um die Beobachtungsinformationen zu empfangen und anzuzeigen, wobei das zentrale Verarbeitungsverfahren aufweist:
einen Speicherschritt zum Speichern, in einem Informationsspeicher (122) für eine Bestimmungsendgerätegruppe, einer assoziativen Verknüpfung zwischen der Sensoreinheit (SU) und einer ersten Gruppe (Gr1), die aus einer Vielzahl von Endgeräten besteht, welche erste Bestimmungsziele sind, an welche die Beobachtungsinformationen über die Person in Verbindung mit dem von der Sensoreinheit (SU) empfangenen Überprüfungsergebnis geschickt werden; und
einen Koordinationsverarbeitungsschritt zum Senden über einen Kommunikator, wenn der Kommunikator von einem ersten Endgerät (TA-1) ein erstes Akzeptanzbenachrichtigungssignal empfängt, das an ein zweites Endgerät (TA-2) gesendet wurde, um den zentralen Prozessor (SV) zu benachrichtigen, dass ein vorbestimmter Vorgang an der Sensoreinheit (SU) von dem ersten Endgerät (TA-1) akzeptiert wird, eines zweiten Akzeptanzbenachrichtigungssignals, das anzeigt, dass der vorbestimmte Vorgang von dem ersten Endgerät akzeptiert wird, wobei das zweite Endgerät zu der ersten Gruppe (Gr1) gehört, zu welcher das erste Endgerät (TA-1) gehört, und sich aber von dem ersten Endgerät (TA-1) unterscheidet.

7. Personenbeobachtungssystem (MS) zur Beobachtung einer Person (Ob), die ein Überwachungsziel ist, wobei das System aufweist:
eine Sensoreinheit (SU) zur Überprüfung der Person
einen zentralen Prozessor (SV) gemäß irgendeinem der Ansprüche 1 bis 5, der kommunikativ mit der Sensoreinheit verbunden ist zur Verwaltung der Beobachtungsinformationen über die Person (Ob) in Verbindung mit einem von der Sensoreinheit empfangenen Überprüfungsergebnis: und
eine Vielzahl von Endgeräten (SP, TA), die kommunikativ mit dem zentralen Prozessor (SV) verbunden sind, um die Beobachtungsinformationen zu empfangen und anzuzeigen.

## Revendications

1. Dispositif de traitement central (SV) destiné à l'utilisation dans un système d'observation de personnes (MS) pour observer une personne comme une cible d'observation (Ob),
le système comprenant une unité de captage (SU) pour examiner la personne, le dispositif de traitement central, qui est relié de manière communicative à l'unité de captage, pour administrer des informations d'observation sur la personne en liaison avec un résultat d'examen reçu de l'unité de captage, et une pluralité de dispositifs terminaux (SP, TA) reliés de manière communicative au dispositif de traitement central pour recevoir et afficher les informations d'observation, le dispositif de traitement central comprenant :
un communicateur (13) configuré pour effectuer une communication ;
un stockage d'informations d'un groupe terminal de destination (122) configuré pour stocker une relation associative entre l'unité de captage et un premier groupe composé d'une pluralité de dispositifs terminaux, qui sont de premières destinations auxquelles les informations d'observation sur la personne en liaison avec le résultat d'examen reçu de l'unité de captage sont envoyées ; et
un processeur de coordination configuré pour inciter le communicateur (13) à envoyer, si le communicateur reçoit un premier signal de notification d'acceptation (C3) d'un premier dispositif terminal (TA-1), lequel signal a été envoyé pour informer le dispositif de traitement central (SV) qu'une opération prédéterminée dans l'unité de captage (SU) est acceptée par le premier dispositif terminal (TA-1), un deuxième signal de notification d'acceptation (C5) à un deuxième dispositif terminal (TA-2), lequel deuxième signal de notification d'acceptation (C5) indique que l'opération prédéterminée est acceptée par le premier dispositif terminal (TA-1), le deuxième dispositif terminal (TA-2) appartenant au premier groupe (Gr1), auquel appartient le premier dispositif terminal (TA-1), mais étant différent du premier dispositif terminal (TA-1).

2. Dispositif de traitement central (SV) selon la revendication 1, dans lequel
le processeur de coordination est configuré pour inciter le communicateur (13) à envoyer en outre un signal d'interdiction d'acceptation (C5) au deuxième dispositif terminal (TA-2), lequel signal indique qu'il est interdit au deuxième dispositif terminal (TA-2) d'accepter l'opération prédéterminée.

3. Dispositif de traitement central (SV) selon la revendication 1 ou la revendication 2, dans lequel
l'unité de captage (SU) comprend au moins un élément parmi un élément de photographie pour générer une image vidéo de la personne (Ob) et un élément d'entrée et de sortie d'un son pour entrer et sortir un son, et l'opération prédéterminée comprend au moins une opération parmi une opération de demande d'une image vidéo pour demander l'image vidéo de la personne générée par l'élément de photographie de l'unité de captage et une opération de demande de communication pour demander une communication vocale via l'élément d'entrée et de sortie d'un son de l'unité de captage.

4. Dispositif de traitement central (SV) selon l'une quelconque des revendications 1 à 3, dans lequel
le processeur de coordination est configuré pour inciter le communicateur à envoyer en outre un signal de communication de réponse pour l'affichage (C5) au deuxième dispositif terminal (TA-2) pour inciter le deuxième dispositif terminal à afficher un message (628), qui informe que le premier dispositif terminal (TA-1) répond.

5. Dispositif de traitement central (SV) selon l'une quelconque des revendications 1 à 4, dans lequel
le stockage d'informations d'un groupe terminal de destination (122) est en outre configuré pour stocker une relation associative entre l'unité de captage (SU) et un deuxième groupe (Gr2) composé d'un autre nombre particulier de dispositifs terminaux (SP, TA), qui sont de deuxièmes destinations auxquelles les informations d'observation sur la personne en liaison avec le résultat d'examen reçu de l'unité de captage (SU) sont envoyées, les informations d'observation étant envoyées au deuxième groupe (Gr2) à côté du premier groupe (Gr1) et
le processeur de coordination est configuré pour inciter le communicateur (13) à envoyer les informations d'observation sur la personne (Ob) en liaison avec le résultat d'examen reçu de l'unité de captage aux dispositifs terminaux (TA, SP) en liaison avec l'unité de captage (SU) et appartenant au deuxième groupe, si le communicateur (13) ne reçoit aucun premier signal de notification d'acceptation jusqu'à ce qu'une période de temps prédéterminée se soit écoulée à partir d'un moment où le communicateur envoie les informations d'observation sur la personne (Ob) en liaison avec le résultat d'examen reçu de l'unité de captage au terminal (TA, SP) en liaison avec l'unité de captage (SU) et appartenant au premier groupe.

6. Procédé de traitement central pour gérer centralement un système d'observation de personnes (MS) pour observer une personne comme une cible d'observation (Ob),
le système comprenant une unité de captage (SU) pour examiner la personne, un dispositif de traitement central (SV), qui est relié de manière communicative à l'unité de captage, pour administrer des informations d'observation sur la personne en liaison avec un résultat d'examen reçu de l'unité de captage, et une pluralité de dispositifs terminaux (SP, TA) reliés de manière communicative au dispositif de traitement central (SV) pour recevoir et afficher les informations d'observation, le procédé de traitement central comprenant :
une étape de stockage pour stocker, dans un stockage d'informations d'un groupe terminal de destination (122), une relation associative entre l'unité de captage (SU) et un premier groupe (Gr1) composé d'une pluralité de dispositifs terminaux, qui sont de premières destinations auxquelles les informations d'observation sur la personne en liaison avec le résultat d'examen reçu de l'unité de captage sont envoyées ; et
une étape de traitement de coordination pour envoyer, via un communicateur, si le communicateur (13) reçoit un premier signal de notification d'acceptation (C3) d'un premier dispositif terminal (TA-1), lequel signal a été envoyé pour informer le dispositif de traitement central (SV) qu'une opération prédéterminée dans l'unité de captage (SU) est acceptée par le premier dispositif terminal (TA-1), un deuxième signal de notification d'acceptation (C5) à un deuxième dispositif terminal (TA-2), lequel deuxième signal de notification d'acceptation (C5) indique que l'opération prédéterminée est acceptée par le premier dispositif terminal (TA-1), le deuxième dispositif terminal (TA-2) appartenant au premier groupe (Gr1), auquel appartient le premier dispositif terminal (TA-1), mais étant différent du premier dispositif terminal (TA-1).

7. Système d'observation de personnes (MS) pour observer une personne (Ob), qui est une cible surveillée, le système comprenant :
une unité de captage (SU) pour examiner la personne :
un dispositif de traitement central (SV) selon l'une quelconque des revendications 1 à 5, qui est relié de manière communicative à l'unité de captage (SU), pour administrer des informations d'observation sur la personne (Ob) en liaison avec un résultat d'examen reçu de l'unité de captage ; et
une pluralité de dispositifs terminaux (SP, TA) reliés de manière communicative au dispositif de traitement central (SV) pour recevoir et afficher les informations d'observation.
